(19) Europäisches Patentamt
European Patent Office
Office européen des brevets

(11) Publication number: **0 142 146 B1**

(12) **EUROPEAN PATENT SPECIFICATION**

(45) Date of publication of patent specification: **31.08.88**

(21) Application number: **84113599.9**

(22) Date of filing: **12.11.84**

(51) Int. Cl.4: **C 07 C 59/90**, C 07 C 69/738, C 07 C 69/76, C 07 C 69/62, C 07 F 7/02, C 07 C 33/46, C 07 C 25/22, C 07 D 261/04 // A61K31/19

# (54) Oxo-analogs of mevinolin-like antihypercholesterolemic agents.

(30) Priority: **14.11.83 US 550707**

(43) Date of publication of application: **22.05.85 Bulletin 85/21**

(45) Publication of the grant of the patent: **31.08.88 Bulletin 88/35**

(84) Designated Contracting States: **AT BE CH DE FR GB IT LI LU NL SE**

(56) References cited:
EP-A-0 052 366
EP-A-0 068 038
GB-A-1 555 831
GB-A-2 055 100
GB-A-2 073 199

(73) Proprietor: **MERCK & CO. INC.**
**126, East Lincoln Avenue P.O. Box 2000**
**Rahway New Jersey 07065-0900 (US)**

(72) Inventor: **Hoffman, William F.**
**740 Wiekel Road**
**Lansdale Pennsylvania 19446 (US)**
Inventor: **Lee, Ta Jyh**
**1921 Supplee Road**
**Lansdale Pennsylvania 19446 (US)**
Inventor: **Stokker, Gerald E.**
**Plymouth Road**
**Gwynedd Valley Pennsylvania 19446 (US)**

(74) Representative: **Abitz, Walter, Dr.-Ing. et al**
**Abitz, Morf, Gritschneder, Freiherr von Wittgenstein Postfach 86 01 09**
**D-8000 München 86 (DE)**

**EP 0 142 146 B1**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid. (Art. 99(1) European patent convention).

Courier Press, Leamington Spa, England.

**Description**

Summary of the Invention

This invention is concerned with novel compounds of structural formula I:

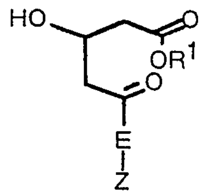

I

wherein Z is a variety of mono- and bi-carbocyclic moieties with various substituents well known to those skilled in the art of 3-hydroxy-3-methylglutaryl Coenzyme A (HMG—CoA) reductase inhibitors useful in the treatment of familial hypercholesterolemia, hyperlipemia and atherosclerosis.

The invention is also concerned with novel processes for the preparation of the novel compounds; pharmaceutical formulations comprising a novel compound as active ingredient; and a method of treating familial hypercholesterolemia, hyperlipemia, and atherosclerosis.

Background of the Invention

Over the past several years a number of structurally related antihypercholesterolemic agents acting by inhibition of HMG—CoA reductase have been reported in the patent literature and elsewhere. The compounds have varied from the natural fermentation products, compactin and mevinolin,

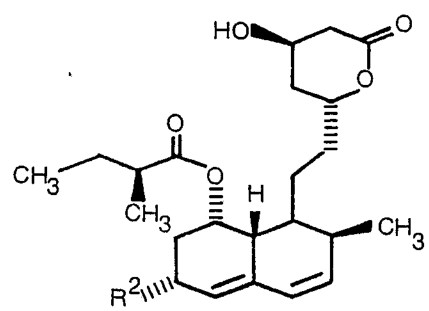

Compactin ($R^2$=H)

Mevinolin ($R^2$=$CH_3$)

(see GB—A—1,555,831, GB—A—2,055,100 and GB—A—2,073,199) to di- and tetrahydro derivatives thereof (see EP—A—0,052,366); to analogs with different esters in the 8-position of the polyhydronaphthalene moiety, to totally synthetic analogs, wherein the polyhydronaphthalene moiety is replaced by substituted mono- and bicyclic aromatics, and biphenyls (see EP—A—0,068,038). But in all instances the active compound included a 4-hydroxytetrahydropyran-2-one ring or the corresponding 3,5-dihydroxy acid, or derivatives thereof, formed by opening the pyranone ring such as:

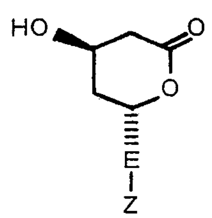

or

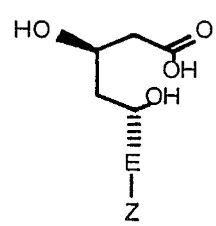

II

4—hydroxytetrahydropyran—2—one

IIa

3,5—dihydroxy—acid

In all of these compounds the 3,5-dihydroxy acid or corresponding lactone moiety is present and the particular stereochemistry depicted is essential for manifestation of the optimum enzyme inhibitory activity.

Now with the present invention there are provided compounds structurally related to those lactones

and dihydroxy acids that do not have the 5-hydroxy functionality, do not form a lactone ring, and are incapable of stereochemical variation at the 5-position of the acid because the 5-carbon is not asymmetric. On the contrary, the 5-carbon carries an oxo function which greatly facilitates the total synthesis of active compounds in that by eliminating one asymmetric center it is unnecessary to separate diastereoisomers or to conduct a stereoselective synthesis to obtain optimum enzyme inhibitory activity. It is believed that structures *I* are reduced *in situ* to generate the "active" inhibitors of structure II or IIa.

The active compounds of this invention are useful in either the racemic form or as the 3(R)-isomer. Those compounds produced by total synthesis are obtained initially as racemates, but may be resolved by standard methods into 3(R)- and 3(S)-isomers. Compounds of Structure I which are synthesized starting from natural fermentation products such as mevinolin and its analogs are obtained as the optically pure 3(R)-isomers.

Detailed Description of the Invention

The novel compounds of this invention have structural formula:

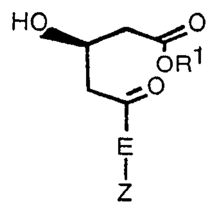

wherein

$R^1$ is

1) hydrogen,
2) $C_{1-4}$alkyl,
3) 2,3-dihydroxypropyl,
4) alkali metal cation, such as $Na^+$, or $K^+$, or
5) ammonium of formula $N^+R^3R^4R^5R^6$

wherein $R^3$, $R^4$, $R^5$ and $R^6$ are independently hydrogen or $C_{1-4}$alkyl or two of $R^3$, $R^4$, $R^5$ and $R^6$ are joined together to form a 5 or 6-membered heterocycle such as pyrrolidino or piperidino with the nitrogen to which they are attached;

E is $-CH_2CH_2-$, $-CH{=}CH-$, or $-(CH_2)_3-$; and

Z is 1)

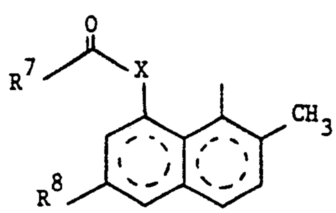

wherein the dotted lines represent all of the possible oxidation states of the bicyclic system such as naphthalene, dihydro-, tetrahydro-, hexahydro-, octahydro-, and decahydronaphthalene;

X is —O— or

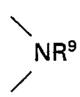

wherein

$R^9$ is H or $C_{1-3}$alkyl;
$R^7$ is $C_{2-8}$alkyl; and
$R^8$ is H or $-CH_3$;

2)

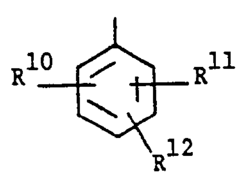

wherein $R^{10}$, $R^{11}$ and $R^{12}$ are independently

a) hydrogen,
b) halogen, such as bromo, chloro or fluoro,
c) $C_{1-4}$alkyl,
d) halo-$C_{1-4}$alkyl,

e) phenyl either unsubstituted or substituted with one or more of
i) $C_{1-4}$alkoxy,
ii) $C_{1-4}$alkyl,
iii) $C_{2-8}$alkanoyloxy, or
iv) halo-$C_{1-4}$alkyl,
v) halo, such as bromo, chloro or fluoro,

f) $OR^{13}$ wherein $R^{13}$ is
i) hydrogen,
ii) $C_{2-8}$alkanoyl,
iii) benzoyl,
iv) phenyl,
v) halophenyl,
vi) phenyl-$C_{1-3}$alkyl, either unsubstituted or substituted with one or more of halogen, $C_{1-4}$ alkoxy, $C_{1-4}$alkyl or halo-$C_{1-4}$alkyl,
vii) $C_{1-9}$alkyl,
viii) cinnamyl,
ix) halo-$C_{1-4}$alkyl,
x) allyl,
xi) $C_{3-6}$cycloalkyl-$C_{1-3}$alkyl,
xii) adamantyl-$C_{1-3}$alkyl,

3) 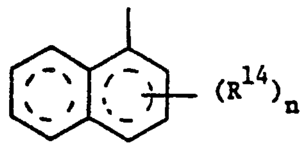

wherein n is 0—2, and $R^{14}$ is halo such as chloro, bromo or fluoro, or $C_{1-4}$ alkyl, and

4) 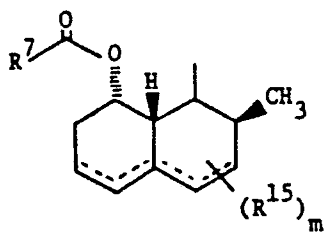

wherein the dotted lines represent possible double bonds there being 0, 1 or 2 double bonds;
m represents 1, 2 or 3; and
$R^{15}$ is
1) methyl,
2) hydroxy,
3) $C_{1-4}$ alkoxy,
4) oxo or
5) halo;

or they have structural formula

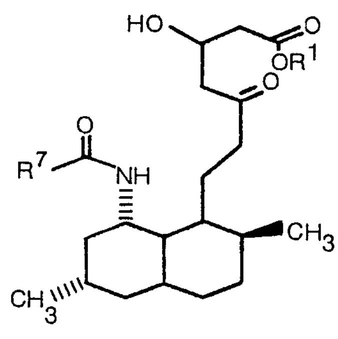

wherein $R^1$ is hydrogen, an alkali metal cation or an ammonium cation and wherein $R^7$ is 4-fluorobenzoyl, 4-tert-butylbenzoyl or 4-fluorophenylacetyl.

Preferred embodiments of the novel compounds are those in which:

$R^1$ is hydrogen, an alkali metal cation or an ammonium cation;

E is —CH=CH— or —$CH_2CH_2$—; and
Z is

1)

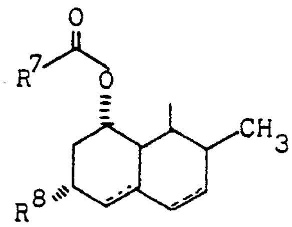

wherein

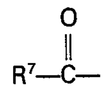

is 2-methylbutyryl or 2,2-dimethylbutyryl;

2)

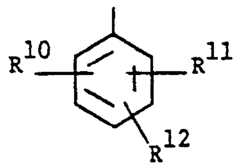

wherein $R^{10}$, $R^{11}$ and $R^{12}$ are independently
- a) halogen,
- b) $C_{1-4}$alkyl,
- c) halo-$C_{1-4}$alkyl,
- d) phenyl with 1 to 3 substituents selected from halo, $C_{1-4}$alkyl or $C_{1-4}$alkoxy,
- e) $OR^{13}$, wherein $R^{13}$ is
  - i) phenyl,
  - ii) halophenyl, or
  - iii) phenyl-$C_{1-3}$ alkyl, either unsubstituted or substituted with one or more of halogen, $C_{1-4}$ alkoxy, $C_{1-4}$ alkyl or halo-$C_{1-4}$ alkyl; or

3)

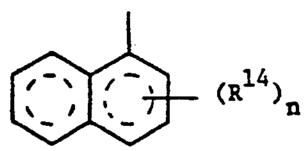

wherein n is 0, 1 or 2 and $R^{14}$ is methyl and the ring system is naphthalene or 5,6,7,8-tetrahydronaphthalene.

One novel process for preparing the novel compounds of this invention is particularly useful when starting with compounds with a pre-formed 4-hydroxytetrahydropyran-2-one moiety or the corresponding 3,5-dihydroxy acid and is illustrated as follows:

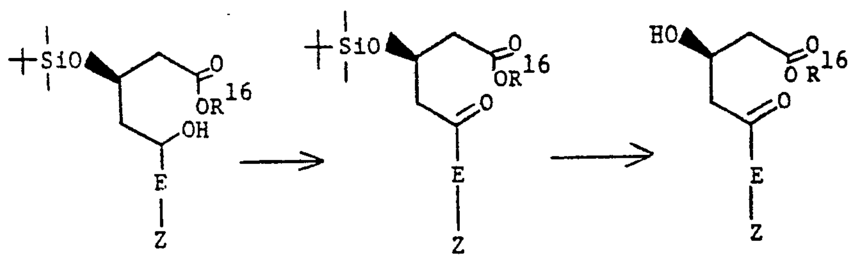

wherein $R^{16}$ is $C_{1-4}$alkyl, especially methyl. After protecting the 4-hydroxyl of the lactone with a dimethyl-*tert*-butylsilyl group and preparing an alkyl ester by known procedures, the resulting 5-hydroxy of the open-chain acid is oxidized to the ketone. Suitable oxiding agents include: pyridinium chlorochromate in a chlorinated alkane such as methylene chloride or chloroform at about 0° to about 25°C for about 1 to 4 hour; oxalyl chloride in dimethylsulfoxide at about −70° to about −40°C for about 0.25 to 0.5 hours; trifluoroacetic anhydride in dimethylsulfoxide at about −70° to −40°C for about 0.25 to 0.5 hour; and pyridinium dichromate in dimethyl formamide at 0° to 25°C for 1 to 8 hours.

The silyl ether group is then hydrolyzed by treatment with acetic acid and tetrabutylammonium fluoride in tetrahydrofuran.

A related procedure is available for preparing compounds of this invention wherein E represents $-CH_2-CH_2-$. It obviates the need for protection of the 3-hydroxy group before oxidizing the 5-hydroxy and is represented as follows:

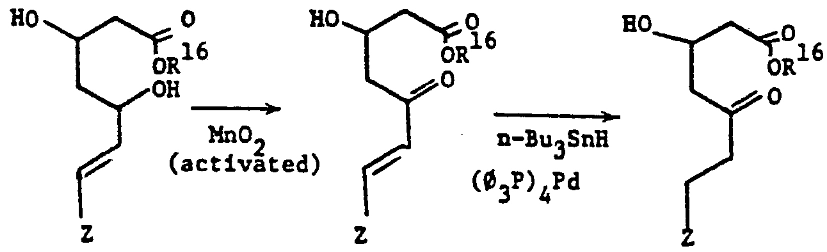


In the first step the dihydroxy compound is treated with activated manganese dioxide in a chlorinated hydrocarbon such as chloroform, methylene chloride, 1,2-dichloroethane or the like at about 0°C to 40°C preferably at ambient temperature for about 15 to 30 hours. The 5-oxo compound produced is then treated with tri-*n*-butyltin hydride and tetrakis(triphenylphosphine)palladium(0) in an ethereal solvent such as ether, THF, 1,2-dimethoxyethane or the like, at about ambient temperature for about 15 to 30 hours.

Alternatively, if the 3-hydroxy-5-oxo-carboxylic acid moiety is being synthesized, the 5-oxo group is realized directly by a process which is another embodiment of this invention and which is exemplified as follows:

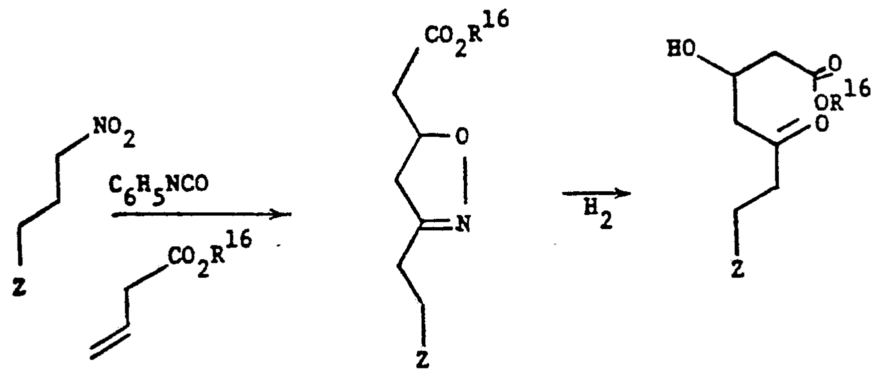


The nitro compound is treated with a $C_{1-4}$alkyl 3-butenoate, preferably methyl 3-butenoate, and an aromatic isocyanate such as p-toluoyl isocyanate, p-chlorophenyl isocyanate, phenyl isocyanate or the like, preferably the latter, and a bit of triethylamine as a catalyst in an inert organic solvent such as toluene, benzene, xylene, or the like at about 15 to 30°C, preferably about room temperature for about 5 to about 24 hours.

The resulting isoxazoline is reduced catalytically with palladium on carbon, platinum oxide or the like in an inert organic solvent such as a $C_{1-3}$alkanol, acetic acid or the like containing a little water in the presence of boric acid at about 15 to 30°C and about 1—2 atmospheres of hydrogen pressure for about 1 to 6 hours.

The ester resulting from either of the foregoing synthetic schemes is readily saponified to the corresponding carboxylic acid salt by treatment with aqueous alkali such as potassium or sodium hydroxide to form the potassium or sodium salt respectively or with a quaternary ammonium hydroxide of formula $HONR^3R^4R^5R^6$ wherein none of the R groups is hydrogen to form the quaternary ammonium salt.

Acidifying any of these salts with a mineral acid results in the formation of the free carboxylic acid.

The acids are readily converted back to salts by treatment with the appropriate base or to esters by treatment with a $C_{1-3}$alkanol in the presence of a catalytic amount of an acid such as hydrogen chloride at about 50 to 100°C for about 3 to 6 hours.

The previously described salts are converted back to esters by treatment with an alkyl halide such as 2,3-dihydroxypropyl iodide in an aprotic solvent such as N,N-dimethylformamide, N-methylpyrrolidone or hexamethylphosphoramide at about 25 to 100°C for about 18 to 36 hours.

Those compounds, wherein Z is of the subtype (4), i.e., in which the polyhydronaphthalene moiety is substituted with hydroxy or oxo, halo or alkoxy are prepared from the corresponding substrate in which the 5-oxo group of the heptenoic acid is already in place. The processes, as applied to the 5-hydroxy analogs or the corresponding lactones, are disclosed in EP application 76601, British patents 2,111,052 and 2,075,013, EP application 74222, and Japanese published applications J58010572 and J57155995. Using those processes there are produced the following compounds:

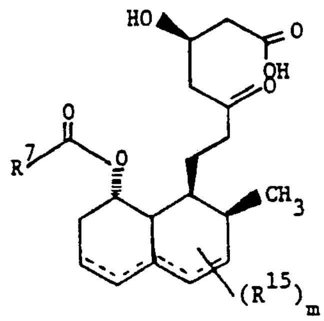

| Double Bonds | $R^7$ | $(R^{15})_m$ |
|---|---|---|
| 3,4:4a,5 | 1-methylpropyl | 6-OH |
| 3,4:4a,5 | 1,1-dimethylpropyl | 6-OH |
| 4,4a | 1-methylpropyl | 3-OH, 5-OH |
| 4,4a | 1,1-dimethylpropyl | 3-OH, 5-OH |
| 4,4a:5,6 | 1-methylpropyl | 3-OH |
| 4,4a:5,6 | 1,1-dimethylpropyl | 3-OH |
| - | 1-methylpropyl | 6-OH |
| - | 1,1-dimethylpropyl | 6-OH |
| - | 1-methylpropyl | 3-OH |
| - | 1,1-dimethylpropyl | 3-OH |
| 4,4a | 1-methylpropyl | 6-OH |
| 4,4a | 1,1-dimethylpropyl | 6-OH |
| 4,4a | 1-methylpropyl | 3-OH |
| 4,4a | 1,1-dimethylpropyl | 3-OH |
| 4a,5 | 1-methylpropyl | 6-OH |
| 4a,5 | 1,1-dimethylpropyl | 6-OH |
| 4a,5 | 1-methylpropyl | 3-OH |
| 4a,5 | 1,1-dimethylpropyl | 3-OH |
| 4,4a | 1-methylpropyl | 3-OH, 5=O |
| 4,4a | 1,1-dimethylpropyl | 3-OH, 5=O |
| 4,4a | 1-methylpropyl | 3=O, 5=O |
| 4,4a | 1,1-dimethylpropyl | 3=O, 5=O |
| - | 1-methylpropyl | 3-OH, 5-OH |
| - | 1,1-dimethylpropyl | 3-OH, 5-OH |
| 4,4a | 1-methylpropyl | 3-Cl, 5-Cl |
| 4,4a | 1,1-dimethylpropyl | 3-Cl, 5-Cl |
| 4,4a | 1-methylpropyl | 3-$OCH_3$, 5-OH |
| 4,4a | 1,1-dimethylpropyl | 3-$OCH_3$, 5-OH |
| 4,4a | 1-methylpropyl | 3-$OC_2H_5$, 5-OH |
| 4,4a | 1,1-dimethylpropyl | 3-$OC_2H_5$, 5-OH |
| 4,4a | 1-methylpropyl | 3-$OC_4H_9$, 5-OH |
| 4,4a | 1,1-dimethylpropyl | 3-$OC_4H_9$, 5-OH |
| 4,4a | 1-methylpropyl | 6-$CH_3$, 3-OH, 5-OH |
| 4,4a | 1,1-dimethylpropyl | 6-$CH_3$, 3-OH, 5-OH |

The novel pharmaceutical composition of this invention comprises at least one of the compounds of formula I in association with a pharmaceutical vehicle or diluent. The pharmaceutical composition can be formulated in a classical manner utilizing solid or liquid vehicles or diluents and pharmaceutical additives of a type appropriate to the mode of desired administration. The compounds can be administered by an oral route, for example, in the form of tablets, capsules, granules or powders, or they can be administered by a parenteral route in the form of injectable preparations.

A typical capsule for oral administration contains active ingredients (25 mg), lactose (75 mg) and magnesium stearate (15 mg). The mixture is passed through a 60 mesh sieve and packed into a No. 1 gelatin capsule.

A typical injectable preparation is produced by asceptically placing 25 mg of a water soluble salt of sterile active ingredient into a vial, asceptically freeze-drying and sealing. For use, the contents of the vial are mixed with 2 ml of physiological saline, to produce an injectable preparation.

The novel method of treating atherosclerosis, familial hypercholesterolemia, or hyperlipemia of this invention comprises administration of an effective antihypercholesterolemic amount of a compound of Formula I to a patient in need of such treatment.

The dose to be administered depends on the unitary dose, the symptoms, and the age and the body weight of the patient. A dose for adults is preferably between 20 and 2,000 mg per day, which can be administered in a single dose or in the form of individual doses from 1—4 times per day.

The componds of this invention also have useful antifungal activities. For example, they may be used to control strains of *Penicillium sp.*, *Aspergillus niger*, *Cladosporium sp.*, *Cochliobolus miyabeorus* and *Helminthosporium cynodnotis*. For those utilities they are admixed with suitable formulating agents, powders, emulsifying agents or solvents such as aqueous ethanol and sprayed or dusted on the plants to be protected.

This invention can be illustrated by the following examples.

## Example 1

7-[2(S),6(R)-Dimethyl-8(S)-(2(S)-methylbutyryloxy)-1,2,6,7,8,8a(R)-hexahydro-1(S)-naphthyl]-3(R)-hydroxy-5-oxoheptanoic acid

*Step A*: Preparation of 6(R)-[2-(8(S)-(2(S)-methylbutyryloxy)-2(S),6(R)-dimethyl-1,2,6,7,8,8a(R)-hexahydronaphthyl-1(S))-ethyl]-4(R)-(dimethyl-*tert*-butylsilyloxy)-3,4,5,6-tetrahydro-2H-pyran-2-one

Mevinolin (4.04 g, 0.01 mol) was dissolved in 25 ml of dry dimethylformamide (DMF) and treated with 2.7 g (0.04 mol) of imidazole and 3 g (0.02 mol) of dimethyl-*tert*-butylsilyl chloride, and the solution was stirred under nitrogen overnight. The mixture was poured into 200 ml of ether, washed with 2 × 50 ml of water, 1 × 25 ml of 1N hydrochloric acid, 1 × 25 ml of saturated aqueous sodium carbonate and 2 × 50 ml of brine, dried over $MgSO_4$ and concentrated to dryness. The residue was chromatographed on a "Still" column of silica gel (6.0 × 17.7 cm, 230—400 mesh) by elution with 45% ether in hexane (V/V) collecting 20 ml fractions. The fractions containing the product (21—52) were combined and concentrated to dryness to give 5.2 of oil.

*Step B*: Preparation of Methyl 7-[2(S), 6(R)-Dimethyl-8(S)-(2(S)-methylbutyryloxy)-1,2,6,7,8,8a(R)-hexahydro-1(S)-naphthyl]-3(R)-(*tert*-butyldimethylsilyloxy)-5(R)-hydroxyheptanoate

The silyl ether from Step A (1.03 g, 0.002 mol) was dissolved in 10 ml of methanol, treated with 2 ml of 1N aqueous sodium hydroxide and the mixture was stirred for 2 hours at room temperature. The methanol was evaporated under reduced pressure and the residue was freed of water by azeotropic distillation of 4 × 10 ml of toluene. The solid residue was dissolved in 5 ml of dry DMF, treated with 300 µl, (0.68 g, 0.0048 mol) of methyl iodide and the mixture was stirred overnight at room temperature. The mixture was poured into 100 ml of ether and washed with 20 ml of water and 20 ml of brine, dired ($MgSO_4$) and concentrated to dryness to give 1.0 g of residue (contained DMF). This material wsa chromatographed on a "Still" column of silica gel (6.0 × 17.7 cm, 230—400 mesh) by elution with 45% ether in hexane (V/V) collecting 20 ml fractions. Fractions 32—50 containing the major component were combined and concentrated to dryness to give 576 mg of oily product.

*Step C*: Preparation of Methyl 7-[2(S), 6(R)-Dimethyl-8(S)-(2(S)-methylbutyryloxy)-1,2,6,7,8,8a(R)-hexahydro-1(S)-naphthyl]-3(R)-(*tert*-butyldimethylsilyloxy)-5-oxoheptanoate

The ester from Step B (586 mg, 0.001 mol) was dissolved in 10 ml of methylene chloride and cooled to 0°C. Pryidine chlorochromate (0.56 g, 0.0026 mol) was added and the stirred mixture was allowed to warm spontaneously over 2 hours. Additional pyridine chlorochromate (224 mg, 0.001 mol) was added and stirring was continued another hour. The methylene chloride was evaporated *in vacuo.* The residue was suspended in 5 ml. ether, placed on top of a 4 × 40 cm column of silica gel (70—230 mesh) and eluted with 40% ether in hexane (V/V) collecting 15 ml fractions. Fractions 10—23 were combined and concentrated to 130 mg. of oily product.

*Step D*: Preparation of Methyl 7-[2(S), 6(R)-Dimethyl-8(S)-(2(S)-methylbutyryloxy)-1,2,6,7,8,8a(R)-hexahydro-1(S)-naphthyl]-3(R)-hydroxy-5-oxoheptanoate

The silyl ether from Step C (230 mg, 0.00024 mol) was dissolved in 5 ml of tetrahydrofuran (THF) and

treated with 54 µl, (0.057 g, 0.00095 mol) of acetic acid and 710 µl (1M in THF, 0.00071 mol) of tetrabutylammonium fluoride ($Bu_4N^+F^-$) and the mixture was stirred overnight at room temperature.

Another 57 µl of acetic acid and 710 µl of $Bu_4N^+F^-$ were added and stirring was continued an additional 24 hours. The mixture was poured into 100 ml of ether and washed with 1 × 5 ml of 1N hydrochloric acid, 1 × 10 ml of saturated aqueous sodium bicarbonate and 2 × 10 ml of brine and dried ($MgSO_4$). Concentration to dryness gave 120 mg of an oil. The oil was chromatographed on a "Still" column of silica gel (1.5 × 17.7 cm, 230—400 mesh) by elution with 5% acetone in methylene chloride (v/v) collecting 5 ml fractions. Fractions 12—20 containing the product were combined and concentrated to dryness to give 53 mg of solid (m.p. 64—66°C). Recrystallization of a sample from hexane gave material with m.p. 67—68°C.

Analysis for $C_{25}H_{38}O_6$ (434.55):
Calc: C, 69.09; H, 8.81.
Found: C, 69.30; H, 9.38.

*Step E*: Preparation of 7-[2(S), 6(R)-Dimethyl-8(S)-(2(S)-methylbutyryloxy)-1,2,6,7,8,8a(R)-hexahydro-1(S)-naphthyl]-3(R)-hydroxy-5-oxoheptanoic acid

The ester from Step D (43 mg, 0.0001 mol) was dissolved in 5 ml of methanol and treated with 2 ml of 0.1N sodium hydroxide (0.0002 mol) and stirred overnight at room temperature. The methanol was evaporated *in vacuo* and the residue was acidified with 1N hydrochloric acid and extracted with ether. The ether extract was washed with 3 × 10 ml of brine and dried over $MgSO_4$. Concentration to dryness provided 36 mg of solid which after recrystallization from ether/hexane had m.p. 102—103°C.

Analysis for $C_{24}H_{36}O_6$ (420.53):
Calc: C, 68.54; H, 8.63.
Found: C, 68.57; H, 8.88.

Employing the procedure substantially as described in Example 1, Steps A through E, but substituting for the mevinolin used in Step A, equimolar amounts of the lactones described in Table I there are produced the corresponding 5-oxo-carboxylic acids, salts, and esters also described in Table I in accordance with the following reaction scheme:

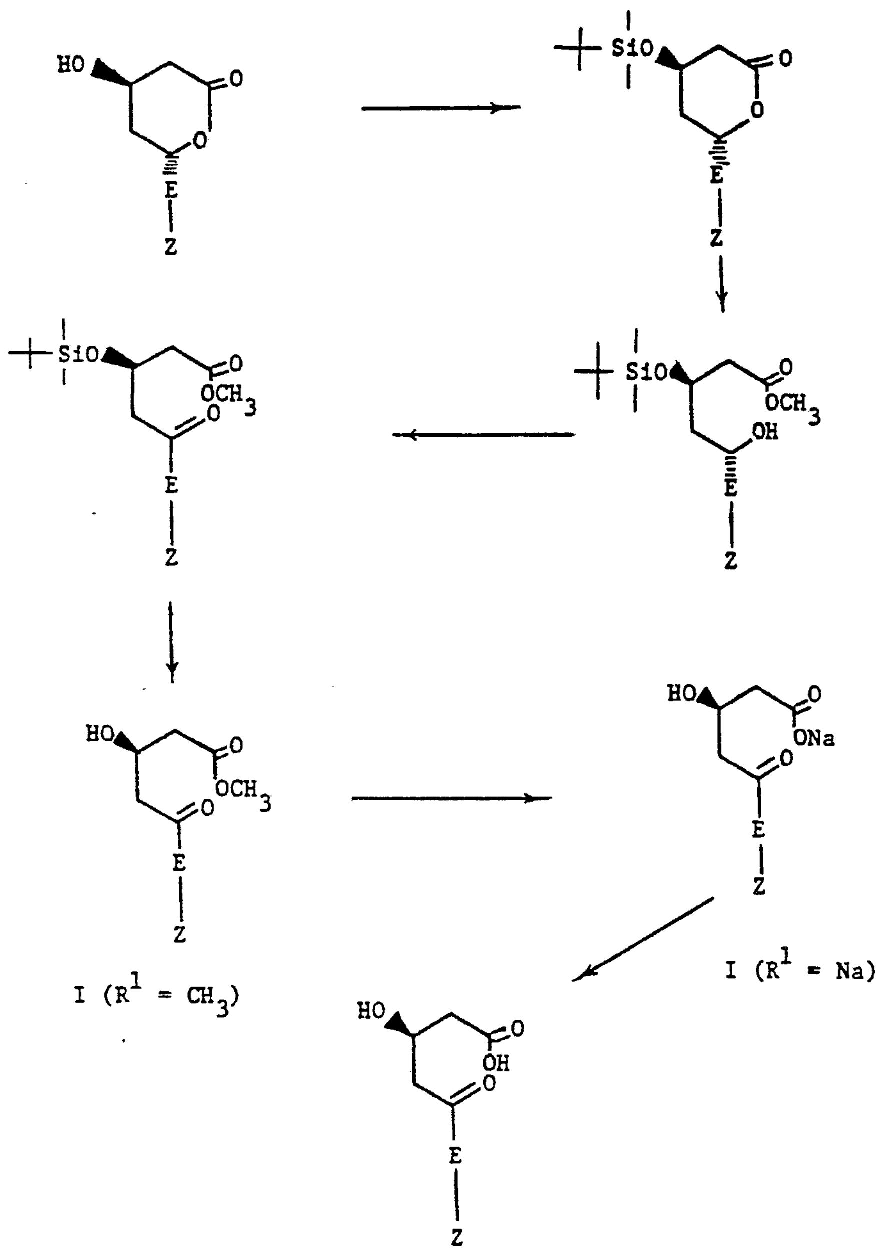

I ($R^1$ = $CH_3$)

I ($R^1$ = Na)

I ($R^1$ = H)

TABLE I

1)

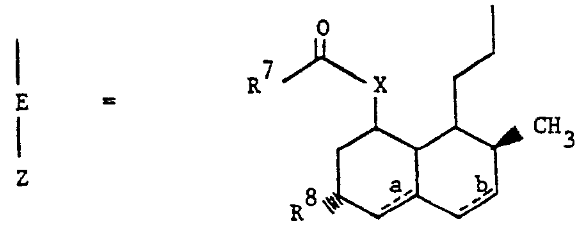

| $R^{7}\overset{\overset{O}{\|}}{C}-$ | $R^8$ | X | a | b |
|---|---|---|---|---|
| 2(S)-methylbutyryl | $-CH_3$ | O | single | double |
| 2(S)-methylbutyryl | $-CH_3$ | O | single | single |
| 2(R)-methylbutyryl | $-CH_3$ | O | double | double |
| 2,2-dimethylbutyryl | $-CH_3$ | O | double | double |
| 2,2-dimethylbutyryl | $-CH_3$ | O | single | double |
| 2,2-dimethylbutyryl | $-CH_3$ | O | single | single |
| acetyl | $-CH_3$ | O | double | double |
| 2(S)-methylbutyryl | H | O | single | single |
| 2,2-dimethylbutyryl | H | O | double | double |
| 2,2-dimethylbutyryl | H | O | single | single |
| 2,2-dimethylbutyryl | $-CH_3$ | NH | single | single |
| 2-methyl-2-ethylbutyryl | $-CH_3$ | NH | single | single |
| 2-methylbutyryl | $-CH_3$ | NH | single | single |
| 4-fluorobenzoyl | $-CH_3$ | NH | single | single |
| 4-fluorophenylacetyl | $-CH_3$ | NH | single | single |
| 4- *tert* -butylbenzoyl | $-CH_3$ | NH | single | single |
| acetyl | $-CH_3$ | NH | double | double |
| acetyl | $-CH_3$ | $NCH_3$ | single | single |
| 2,2-dimethylbutyryl | $-CH_3$ | $NCH_3$ | single | single |
| 2,2-dimethylbutyryl | $-CH_3$ | NH | double | double |

(2)

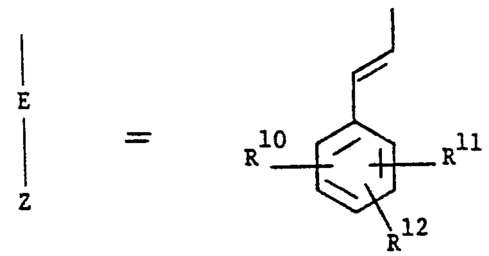

| $R^{10}$ | $R^{11}$ | $R^{12}$ |
|---|---|---|
| 6-(4-fluoro-3-methylphenyl)- | 2-methyl | 4-methyl |
| 6-(4-fluorophenyl)- | 2-chloro | 4-chloro |
| 6-(4-chlorophenyl)- | 2-chloro | 4-chloro |
| 6-(3,4-dichlorophenyl)- | 2-chloro | 4-chloro |
| 6-(4-fluoro-3-methylphenyl)- | 2-chloro | 4-chloro |
| 6-(3,4-dichlorophenyl)- | 2-methyl | 4-methyl |
| 6-(3,5-dimethylphenyl)- | 2-chloro | 4-chloro |
| 6-(3,4-dichlorophenyl)- | 2-methyl | 5-methyl |
| 6-(4-fluorophenyl)- | 2-methyl | 4-methyl |
| 6-(4-fluoro-3-methylphenyl)- | 2-methyl | 4-chloro |
| 6-(4-fluorobenzyloxy) | 2-chloro | 4-chloro |
| 6-(4-fluoro-3-methylphenyl) | 2-chloro | 4-methyl |

3)

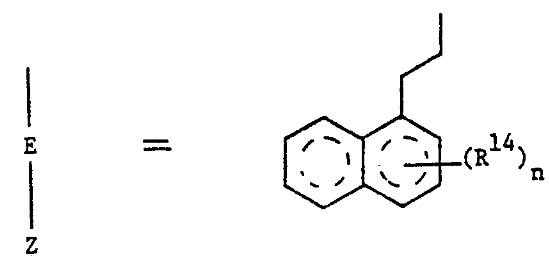

| n | $R^{14}$ | |
|---|---|---|
| 1 | 2-methyl | naphthyl |
| 0 | - | naphthyl |
| 2 | 2,6-dimethyl | naphthyl |
| 1 | 2-methyl | 5,6,7,8-tetra-hydronaphthyl |

Example 2

7-(4'-Fluoro-3,3',5-trimethyl-[1,1'-biphenyl]-2-yl)-3-hydroxy-5-oxoheptanoic acid

*Step A*: Preparation of Methyl 3-(4'-Fluoro-3,3',5-trimethyl-[1,1'biphenyl]-2-yl)propionate

A solution of 1.716 g (13 mmol) of dimethyl malonate in 5 ml of DMF was added dropwise to a stirred suspension of sodium hydride (50% oil dispersion, 0.624 g, 13 mmol) in 15 ml of DMF and stirring was continued under nitrogen for 0.5 hour. The mixture was treated with ice bath cooling, with a solution of 3.1 g (11.8 mmol) of 2-chloromethyl-4'-fluoro-3,3',5-trimethyl-1,1'-biphenyl in 10 ml of DMF. The resulting mixture was stirred at 0°C for 10 minutes, at room temperature for 0.5 hour, and heated on a steam bath for 1 hour. Sodium chloride (0.759 g, 13 mmol) and 0.234 ml (13 mmol) of water were added to the reaction mixture and it was heated at reflux for 16 hours. The reaction mixture was cooled, poured into cold water and extracted with ether twice. The combined extracts were washed with dilute hydrochloric acid, dried

over $MgSO_4$, filtered and concentrated to dryness *in vacuo* to give 3.42 (11.38 mmol, 96%) of the desired product as a brown oil which was used directly in the next step without purification.

nmr ($CDCl_3$) δ: 2.27 (6H, a methyl singlet and a methyl doublet), 2.3 (2H, m), 2.34 (3H, s), 2.9 (2H, m), 3.60 (3H, s), 6.84 (H, bs), 7.1—7.2 (4H, m).

*Step B*: Preparation of 3-(4′-fluoro-3,3′,5-trimethyl[1,1′-biphenyl]-2-yl)propanol

A solution of 3.42 g (11.4 mmol) of the ester from Step A in 25 ml of ether was added dropwise to a stirred suspension of 0.38 g (10 mmol) of lithium aluminum hydride in 75 ml of ether at 0°C under nitrogen. After completion of the addition, the mixture was stirred at room temperature for 15 minutes, refluxed for 1 hour, cooled in ice and treated with successive additions of 0.4 ml of water, 0.35 ml of 20% (w/v) aqueous sodium hydroxide and 1.1 ml of water. The resulting mixture was stirred at 0°C for 0.5 hour, treated wih anhydrous $MgSO_4$, stirred for 15 minutes and filtered. The filtrate was concentrated *in vacuo* to give 3.08 g (11.3 mmol) (99%) of pale yellow oily product which was used directly in the next step without purification.

nmr ($CDCl_3$) δ: 1.45—1.7 (2H, m), 2.25 (6H, s), 2.33 (3H, s), 2.45—2.7 (2H, m), 3.45 (2H, t, J=6Hz), 6.85 (H, bs), 6.95—7.2 (4H, m).

*Step C*: Preparation of 2-(3-Bromopropyl)-4′-fluoro-3,3′,5-trimethyl-1,1′-biphenyl

A solution of 1.08 g (4 mmol) of $PBr_3$ in 10 ml of ether was added dropwise to a stirred solution of 3.08 g (11.3 mmol) of the alcohol from Step B in 40 ml of ether at 0°C. The mixture was stirred at room temperature for 1 hour, refluxed for 0.5 hour, cooled to room temperature, poured into ice water and extracted with ether. The extract was washed with water and saturated aqueous sodium bicarbonate, dried over $MgSO_4$, filtered and evaporated to dryness *in vacuo*. The residue was purified by flash chromatography on silica gel (230—400 mesh) by elution with methylene chloride/hexane (1:3, v/v). Combination and evaporation of the appropriate fractions gave the desired bromide as a pale yellow oil, (1.9 g, 5.67 mmol, 48% overall Steps A, B and C).

nmr ($CDCl_3$) δ: 1.7—2.0 (2H, m), 2.27 (6H, a methyl singlet and a methyl doublet), 2.35 (3H, s), 2.55—2.8 (2H, m), 3.23 (2H, t, J=6Hz), 6.85 (H, bs), 6.95—7.2 (4H, m).

*Step D*: Preparation of 4′-Fluoro-3,3′,5-trimethyl-2-(3-nitropropyl)-1,1′-biphenyl

A solution of 1.90 g (5.66 mmol) of the bromopropyl compound from Step C in 5 ml of ether was added to a stirred suspension of 1.31 g (8.5 mmol) of silver nitrite in 5 ml of ether at 0°C. The resulting mixture was stirred under nitrogen at 0°C for 7 hours, warmed to room temperature and stirred for an additional 16 hours. Another 1.0 g of silver nitrite was added and stirring was continued for another 20 hours.

The reaction mixture was filtered and the filtrate was concentrated to leave a residue which was purified by flash chromatography on silica gel (230—400 mesh) by elution with methylene chloride/hexane (1:4, v/v) to give, first, the recovered starting bromide, then the desired product, (0.64 g, 2.12 mmol, 78%).

nmr ($CDCl_3$) δ: 1.8—2.2 (2H, m), 2.30 (6H, a methyl singlet and a methyl doublet), 2.33 (3H, s), 2.5—2.7 (2H, m), 4.18 (2H, t, J=6Hz), 6.88 (H, bs), 7.0—7.2 (4H, m). IR (neat) 1550, 1500 $cm^{-1}$.

*Step E*: Preparation of Methyl 3-[2-(4′-fluoro-3,3′,5-trimethyl[1,1′-biphenyl]-2-yl)ethyl]-4,5-dihydro-5-isoxazoleacetate

A solution of 0.1 g (1.0 mmol) of methyl 3-butenoate and 0.174 ml (1.6 mmol) of phenyl isocyanate in 1 ml of toluene was added with stirring to a solution of 0.240 g (0.8 mmol) of the nitropropyl compound from Step D and 2 drops of triethylamine in 1 ml of toluene. The resulting mixture was stirred at room temperature for 3 hours. Additional quantities of methyl 3-butenoate (0.1 ml), triethylamine (0.1 ml) and phenyl isocyanate (0.15 ml) were added successively and stirring was continued overnight (18 hours). The mixture was filtered and the filtrate was concentrated *in vacuo* to a residue which was purified by flash chromatography on silica gel (230—400 mesh), first being eluted with methylene chloride to remove the impurities. Continued elution with acetone/methylene chloride (1:50, v/v) gave the desired product (0.218 g, 0.57 mmol, 71%) as a pale viscous oil.

nmr ($CDCl_3$) δ: 2.28 (6H, s), 2.32 (3H, s), 2.2—3.0 (6H, m), 3.70 (3H, s), 4.6—5.0 (H, m), 6.85 (H, bs), 7.0—7.2 (4H, m). IR (neat) 1735 $cm^{-1}$.

| | |
|---|---|
| Analysis calculated for $C_{23}H_{26}FNO_3$: | C, 72.04; H, 6.83; N, 3.65. |
| Found: | C, 72.35; H, 6.99; N, 3.88. |

*Step F*: Preparation of Methyl 7-(4′-fluoro-3,3′,5-trimethyl-[1,1′-biphenyl]-2-yl)-3-hydroxy-5-oxoheptanoate

A mixture of 0.1 g (0.26 mmol) of the isoxazoline from Step E, 50 mg of 10% palladium on carbon catalyst and 48 mg (0.78 mmol) of boric acid in 3 ml of methanol and 0.3 ml of water was stirred under hydrogen (1 atmosphere) at room temperature for 2.5 hours. The mixture was filtered and the filtrate was poured into brine and extracted with ether. The ethereal extract was washed with 5% (w/v) aqueous sodium bicarbonate solution, dried ($MgSO_4$), filtered and evaporated to dryness to give 92 mg (0.23 mmol, 89%) as a pale yellow oil.

nmr ($CDCl_3$) δ: 2.30 (6H, a methyl singlet and a methyl doublet), 2.33 (3H, s), 2.35—2.5 (6H, m), 2.75—2.85 (2H, m), 3.30 (H, d), 3.70 (3H, s), 4.37 (H, m), 6.83 (H, bs), 6.95—7.1 (4H, m). IR (neat) 3450, 1710 $cm^{-1}$.

*Step G*: Preparation of 7-(4′-fluoro-3,3′,5-trimethyl-[1,1′-biphenyl]-2-yl)-3-hydroxy-5-oxoheptanoic acid

Employing the procedure substantially as described in Example 1, Step E, the ester from Step G of this Example 2 is saponified to the subject 5-keto acid.

Employing the procedure substantially as described in Example 2, Steps A through G, but substituting for the chloromethylbiphenyl employed in Step A thereof, equimolar amounts of the chloromethyl compounds described in Table II, there are produced the 5-keto esters, salts and acids also described in Table II in accordance with the following reaction sequence:

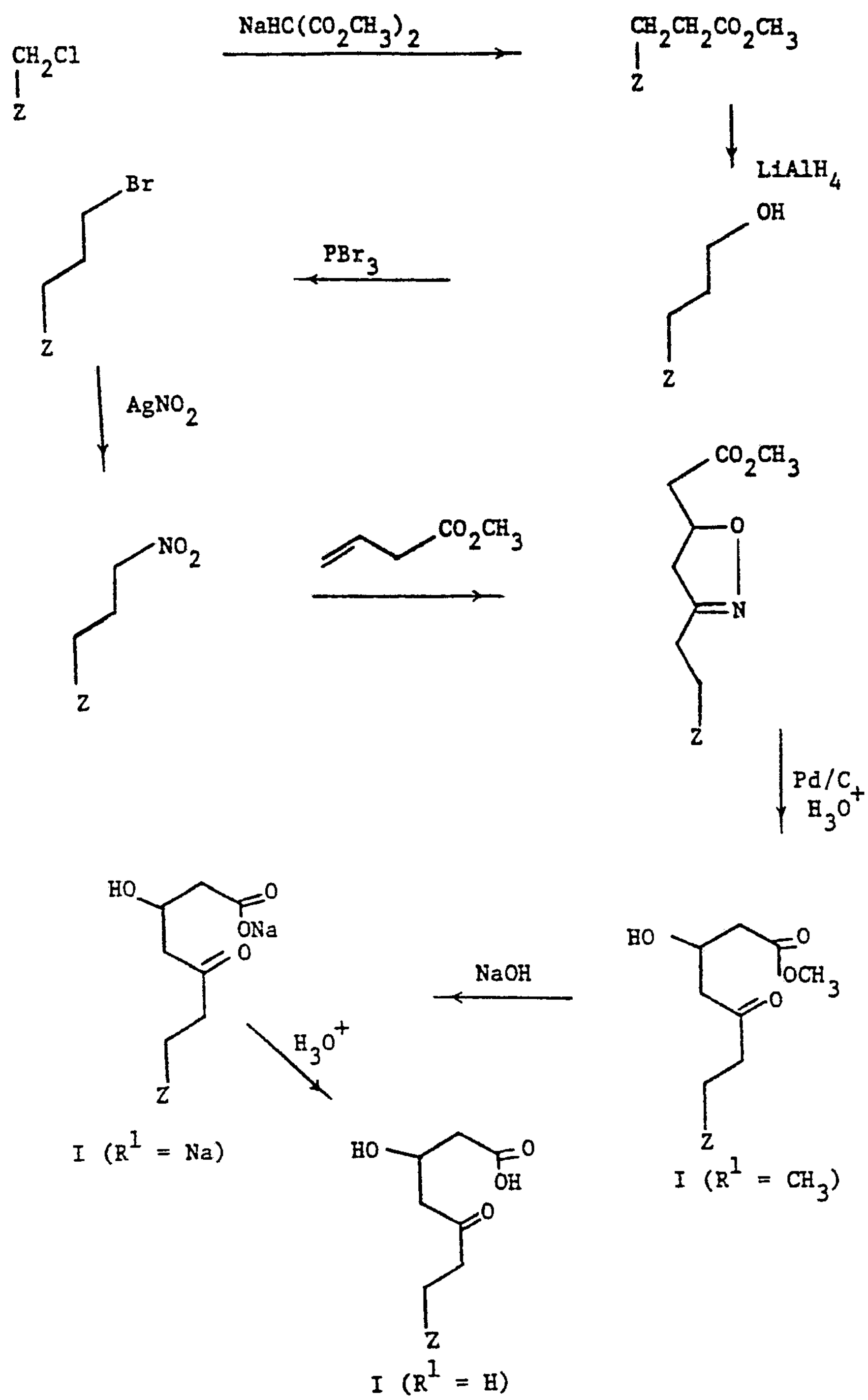

TABLE II

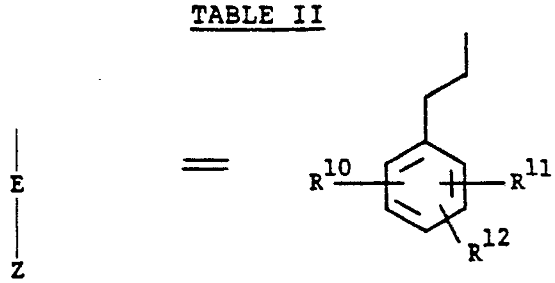

| $R^{10}$ | $R^{11}$ | $R^{12}$ |
|---|---|---|
| 6-(4-fluorophenyl)- | 2-chloro | 4-chloro |
| 6-(4-chlorophenyl)- | 2-chloro | 4-chloro |
| 6-(3,4-dichlorophenyl)- | 2-chloro | 4-chloro |
| 6-(4-fluoro-3-methylphenyl)- | 2-chloro | 4-chloro |
| 6-(3,4-dichlorophenyl)- | 2-methyl | 4-methyl |
| 6-(3,4-dimethylphenyl)- | 2-chloro | 4-chloro |
| 6-(3,4-dichlorophenyl)- | 2-methyl | 5-methyl |
| 6-(4-fluorophenyl)- | 2-methyl | 4-methyl |
| 6-(4-fluoro-3-methylphenyl)- | 2-methyl | 4-chloro |
| 6-(4-fluorobenzyloxy) | 2-chloro | 4-chloro |
| 6-(4-fluoro-3-methylphenyl)- | 2-chloro | 4-methyl |

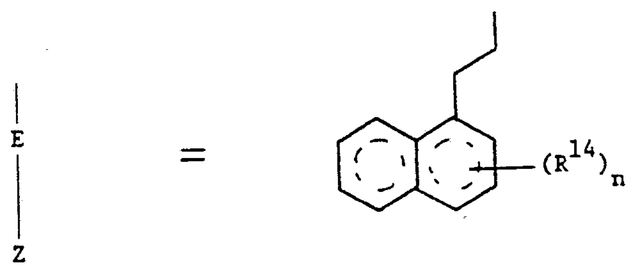

| n | $R^{14}$ | |
|---|---|---|
| 1 | 2-methyl | naphthyl |
| 0 | - | naphthyl |
| 2 | 2,6-dimethyl | naphthyl |
| 1 | 2-methyl | 5,6,7,8-tetra-hydronaphthyl |

Example 3

7-(2,4-Dichlorophenyl)-3-hydroxy-5-oxoheptanoic acid

*Step A*: Preparation of Methyl 7-(2,4-Dichlorophenyl)-3-hydroxy-5-oxo-6-heptenoate

Activated manganese dioxide (40 g) was added to a solution of methyl 7-(2,4-dichlorophenyl)-3,5-dihydroxy-6-heptenoate (6.8 g, 21.3 mmol) in chloroform (600 mL) and the black suspension was vigorously stirred at ambient temperature for 20 hours. After filtration and evaporation of the solvent the residual amber oil (4.5 g, 1 major spot on TLC with $F_f$ 0.61 on Whatman MK6F silica using $CHCl_3$—MeOH,

19:1 as eluent) was chromatographed on a Still column to obtain the product (3.9 g, 58%) as a pale yellow oil which solidified on standing, m.p. 77—79°C;

nmr ($CDCl_3$) δ: 2.57 (2H, d, J=6Hz, —$CH_2CO_2$—), 2.93 (2H, d, J=6Hz, —$CH_2$—CO—), 3.70 (3H, s, —$CO_2CH_3$), 4.4—4.8 (H, m, —*CH*(OH)—), 6.67 (H, d, J=16 Hz, =CH—CO), 7.1—7.7 (3H, m. ArH), 7.93 (H, d, J=16 Hz, =CH).

Analysis for $C_{14}H_{14}Cl_2O_4$.
Calc: C, 53.02; H, 4.45.
Found: C, 53.25; H, 4.50.

*Step B*: Preparation of Methyl 7-(2,4-Dichlorophenyl)-3-hydroxy-5-oxoheptanoate

Tributyltin hydride (450 μL, 1.7 mmol) was added dropwise over 1½ hours to a stirred solution of the ene-one ester from Step A (320 mg, 1 mmol) and tetrakis(triphenylphosphine)palladium(O) (35 mg, 0.03 mmol) in dry THF (5 mL) at ambient temperature under $N_2$. After standing at 20°C overnight the light-brown solution was distributed between water (100 mL) and ether (150 mL). The organic layer was separated and washed with water (2 × 100 mL), dried and evaporated. The residual oil (1 major spot on TLC with $R_f$ 0.39 vis-a-vis 0.35 for the starting ene-one ester on Whatman MK6F silica using $CHCl_3$—NeOH; 99:1 as eluent) was chromatographed on a Still column to obtain the product (260 mg, 81%) as a pale amber gum;

nmr ($CDCl_3$) δ: 2.5—2.525 (2H, m, —$CH_2CO_2$—), 2.57—2.73 (2H, m, —CO*CH*$_2$C(OH)—), 2.77 (2H, t, J=7.5 Hz, AR—*CH*$_2$$CH_2$CO—), 2.98 (2H, t, J=7.5 Hz, AR—*CH*$_2$$CH_2$CO—), 3.71 (3H, s, —$CO_2CH_3$), 4.45—4.51 (H, m, —*CH*(OH)—).

Analysis for $C_{14}H_{16}Cl_2O_4$
Calc: C, 52.68; H, 5.05.
Found: C, 52.47; H, 5.20.

*Step C*: Preparation of 7-(2,4-dichlorophenyl)-3-hydroxy-5-oxoheptanoic acid

Employing the procedure substantially as described in Example 1, Step E, the ester from Step B of this Example 3 is saponified to the subject 5-oxo acid.

**Claims for the Contracting States: BE CH DE FR GB IT LI LU NL SE**

1. A compound of structural formula:

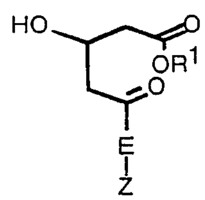

wherein:

$R^1$ is
1) hydrogen,
2) $C_{1-4}$alkyl,
3) 2,3-dihydroxypropyl,
4) alkali metal cation, or
5) ammonium of formula

$$\overset{+}{N}R^3R^4R^5R^6$$

wherein $R^3$, $R^4$, $R^5$ and $R^6$ are independently hydrogen or $C_{1-4}$alkyl or two of $R^3$, $R^4$, $R^5$ and $R^6$ are joined together to form a 5- or 6-membered heterocycle with the nitrogen to which they are attached;

E is —$CH_2CH_2$, —CH=CH—, or —$(CH_2)_3$—; and

Z is

1)

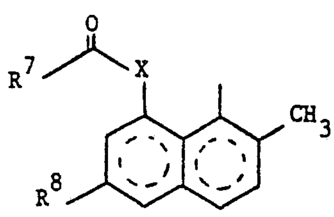

wherein the dotted lines represent all of the possible oxidation states of the bicyclic system, X is —O— or —$NR^9$ wherein $R^9$ is hydrogen or $C_{1-3}$alkyl;

$R^7$ is $C_{2-8}$alkyl; and

$R^8$ is hydrogen or $—CH_3$;

2)

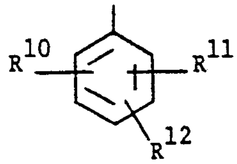

wherein $R^{10}$, $R^{11}$ and $R^{12}$ are independently
a) hydrogen,
b) halogen, such as bromo, chloro or fluoro,
c) $C_{1-4}$alkyl,
d) halo-$C_{1-4}$alkyl,
e) phenyl either unsubstituted or substituted with one or more of
i) $C_{1-4}$alkoxy,
ii) $C_{1-4}$alkyl,
iii) $C_{2-8}$alkanoyloxy,
iv) halo-$C_{1-4}$alkyl, or
v) halo,
f) $OR^{13}$ wherein $R^{13}$ is
i) hydrogen,
ii) $C_{2-8}$alkanoyl,
iii) benzoyl,
iv) phenyl,
v) halophenyl,
vi) phenyl-$C_{1-3}$alkyl, either unsubstituted or substituted with one or more of halogen, $C_{1-4}$alkoxy, $C_{1-4}$alkyl or halo-$C_{1-4}$alkyl,
vii) $C_{1-9}$alkyl,
viii) cinnamyl,
ix) halo-$C_{1-4}$alkyl,
x) allyl,
xi) $C_{3-6}$cycloalkyl-$C_{1-3}$alkyl, or
xii) adamantyl-$C_{1-3}$alkyl;

3)

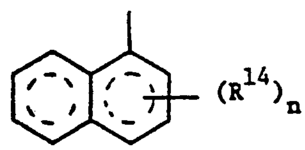

wherein n is 0—2 and $R^{14}$ is halo or $C_{1-4}$ alkyl; and

4)

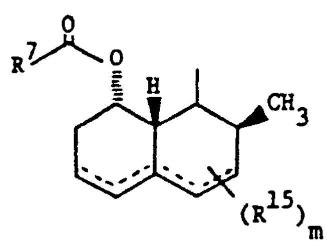

wherein the dotted lines represent possible double bonds there being 0, 1 or 2 double bonds;
m represents 1, 2 or 3; and
$R^{15}$ is
1) methyl,
2) hydroxy,
3) $C_{1-4}$ alkoxy,
4) oxo, or
5) halo.

2. A compound of structural formula

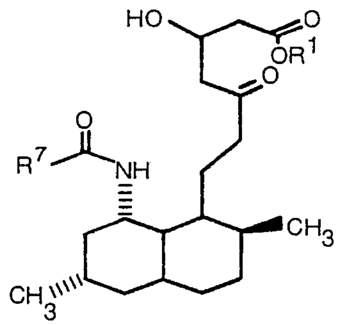

wherein $R^1$ is hydrogen, an alkali metal cation or an ammonium cation and wherein $R^7$ is 4-fluorobenzoyl, 4-tert-butylbenzoyl or 4-fluorophenylacetyl.

3. The compound of Claim 1 wherein:

$R^1$ is hydrogen, an alkali metal cation or an ammonium cation;

E is $-CH{=}CH-$ or $-CH_2CH_2-$; and

Z is

1)

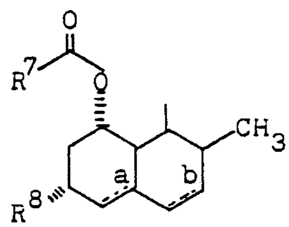

wherein

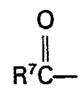

is 2(S)-methylbutyryl or 2,2-dimethylbutyryl;

2)

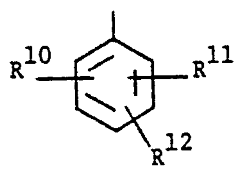

wherein $R^{10}$, $R^{11}$ and $R^{12}$ are independently

a) halogen,

b) $C_{1-4}$alkyl,

c) halo-$C_{1-4}$alkyl,

d) phenyl with 1 to 3 substituents selected from halo, $C_{1-4}$alkyl or $C_{1-4}$alkoxy,

e) $OR^{13}$, wherein $R^{13}$ is

i) phenyl,

ii) halophenyl, or

iii) phenyl-$C_{1-3}$ alkyl, either unsubstituted or substituted with one or more of halogen, $C_{1-4}$ alkoxy, $C_{1-4}$ alkyl or halo-$C_{1-4}$ alkyl; or

3)

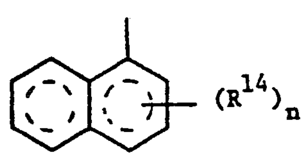

wherein n is 0, 1 or 2, and $R^{14}$ is methyl, and the ring system is naphthyl, or 5,6,7,8-tetrahydronaphthyl.

4. The compound of Claim 1 selected from:

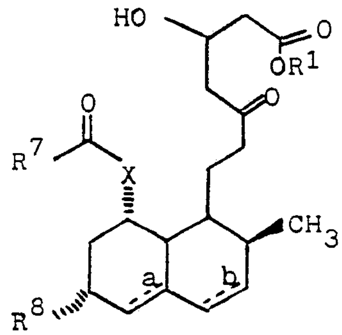

wherein $R^1$ is hydrogen, an alkali metal cation or an ammonium cation and wherein $R^7CO$—, $R^8$, X a and b have the following meanings:

| $R^7C(=O)$– | $R^8$ | X | a* | b |
|---|---|---|---|---|
| 2(S)-methylbutyryl | —$CH_3$ | O | single | double |
| 2(S)-methylbutyryl | —$CH_3$ | O | single | single |
| 2(R)-methylbutyryl | —$CH_3$ | O | double | double |
| 2,2-dimethylbutyryl | —$CH_3$ | O | double | double |
| 2,2-dimethylbutyryl | —$CH_3$ | O | single | double |
| 2,2-dimethylbutyryl | —$CH_3$ | O | single | single |
| acetyl | —$CH_3$ | O | double | double |
| 2(S)-methylbutyryl | H | O | double | double |
| 2(S)-methylbutyryl | H | O | single | single |
| 2,2-dimethylbutyryl | H | O | double | double |
| 2,2-dimethylbutyryl | H | O | single | single |
| 2,2-dimethylbutyryl | —$CH_3$ | NH | single | single |
| 2-methyl-2-ethylbutyryl | —$CH_3$ | NH | single | single |
| 2-methylbutyryl | —$CH_3$ | NH | single | single |
| acetyl | —$CH_3$ | NH | double | double |
| acetyl | —$CH_3$ | $NCH_3$ | single | single |
| 2,2-dimethylbutyryl | —$CH_3$ | $NCH_3$ | single | single |
| 2,2-dimethylbutyryl | —$CH_3$ | NH | double | double |

* When a = single bond, the rings are *trans*-fused.

5. The compound of claim 3 selected from:

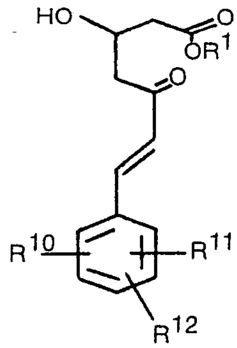

| $R^{10}$ | $R^{11}$ | $R^{12}$ |
|---|---|---|
| 6-(4-fluoro-3-methylphenyl)- | 2-methyl | 4-methyl |
| 6-(4-fluorophenyl)- | 2-chloro | 4-chloro |
| 6-(4-chlorophenyl)- | 2-chloro | 4-chloro |
| 6-(3,4-dichlorophenyl) | 2-chloro | 4-chloro |
| 6-(4-fluoro-3-methylphenyl) | 2-chloro | 4-chloro |
| 6-(3,4-dichlorophenyl) | 2-methyl | 4-methyl |
| 6-(3,5-dimethylphenyl)- | 2-chloro | 4-chloro |
| 6-(3,4-dichlorophenyl)- | 2-methyl | 5-methyl |
| 6-(4-fluorophenyl) | 2-methyl | 4-methyl |
| 6-(4-fluoro-3-methylphenyl)- | 2-methyl | 4-chloro |
| 6-(4-fluorobenzyloxy) | 2-chloro | 4-chloro |
| 6-(4-fluoro-3-methylphenyl)- | 2-chloro | 4-methyl |

6. The compound of claim 3 selected from:

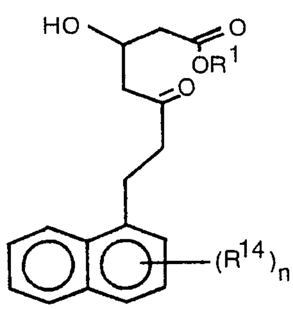

| n | $R^{14}$ | 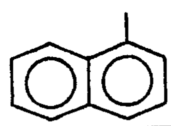 |
|---|---|---|
| 1 | 2-methyl | naphthyl |
| 0 | — | naphthyl |
| 2 | 2,6-dimethyl | naphthyl |
| 1 | 2-methyl | 5,6,7,8-tetrahydronaphthyl |

7. An antihypercholesterolemic pharmaceutical composition comprising a pharmaceutical carrier and an effective antihypercholesterolemic amount of a compound as claimed in Claim 1 or 2.

8. The formulation of Claim 7 wherein the antihypercholesterolemic compound is as claimed in Claim 3.

9. The formulation of Claim 8 wherein the antihypercholesterolemic compound is as claimed in Claims 4, 5 or 6.

10. A process for the preparation of a compound of structural formula:

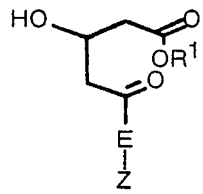

wherein $R^1$, E and Z have the meanings of $R^1$, E and Z 1), 2) and 3) in claim 1, which comprises treating a compound of structural formula:

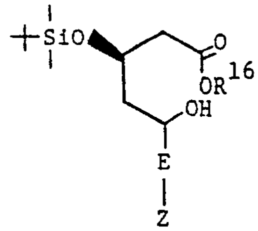

wherein $R^{16}$ is $C_{1-4}$alkyl, with an oxidizing agent to produce the compound of structural formula:

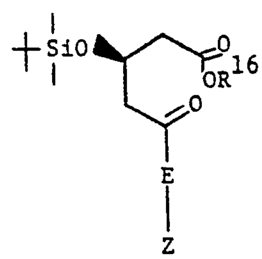

followed by desilylation to produce the compound of structural formula:

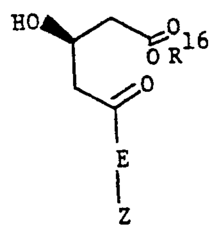

followed by treatment with alkali to produce the product wherein $R^{16}$ is an alkali metal cation, followed by acidification to produce the compound wherein $R^{16}$ is a hydrogen ion.

**Claims for the Contracting State: AT**

1. A process for the preparation of a compound of structural formula:

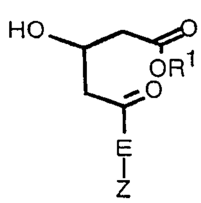

wherein:

$R^1$ is

1) hydrogen,

2) $C_{1-4}$alkyl,
3) 2,3-dihydroxypropyl,
4) alkali metal cation, or
5) ammonium of formula

$$\overset{+}{N}R^3R^4R^5R^6$$

wherein $R^3$, $R^4$, $R^5$ and $R^6$ are independently hydrogen or $C_{1-4}$alkyl or two of $R^3$, $R^4$, $R^5$ and $R^6$ are joined together to form a 5- or 6-membered heterocycle with the nitrogen to which they are attached;

E is $-CH_2CH_2$, $-CH{=}CH-$, or $-(CH_2)_3-$; and

Z is

1) 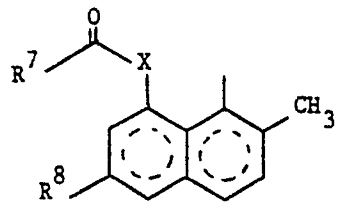

wherein the dotted lines represent all of the possible oxidation states of the bicyclic system, X is —O— or $>NR^9$

wherein $R^9$ is hydrogen or $C_{1-3}$alkyl;

$R^7$ is $C_{2-8}$alkyl; and

$R^8$ is hydrogen or $-CH_3$;

2) 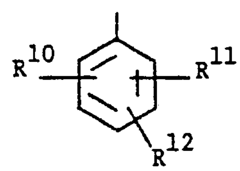

wherein $R^{10}$, $R^{11}$ and $R^{12}$ are independently
a) hydrogen,
b) halogen, such as bromo, chloro or fluoro,
c) $C_{1-4}$alkyl,
d) halo-$C_{1-4}$alkyl,
e) phenyl either unsubstituted or substituted with one or more of
   i) $C_{1-4}$alkoxy,
   ii) $C_{1-4}$alkyl,
   iii) $C_{2-8}$alkanoyloxy, or
   iv) halo-$C_{1-4}$alkyl,
   v) halo,
f) $OR^{13}$ wherein $R^{13}$ is
   i) hydrogen,
   ii) $C_{2-8}$alkanoyl,
   iii) benzoyl,
   iv) phenyl,
   v) halophenyl,
   vi) phenyl-$C_{1-3}$alkyl, either unsubstituted or substituted with one or more of halogen, $C_{1-4}$alkoxy, $C_{1-4}$alkyl or halo-$C_{1-4}$alkyl,
   vii) $C_{1-9}$alkyl,
   viii) cinnamyl,
   ix) halo-$C_{1-4}$alkyl,
   x) allyl,
   xi) $C_{3-6}$cycloalkyl-$C_{1-3}$alkyl, or
   xii) adamantyl-$C_{1-3}$alkyl;

3) 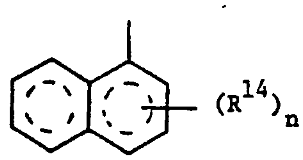

wherein n is 0—2 and $R^{14}$ is halo or $C_{1-4}$ alkyl, which comprises treating a compound of structural formula:

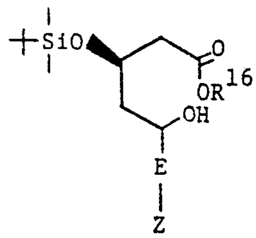

wherein $R^{16}$ is $C_{1-4}$alkyl, with an oxidizing agent to produce the compound of structural formula:

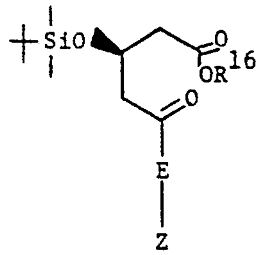

followed by desilylation to produce the compound of structural formula:

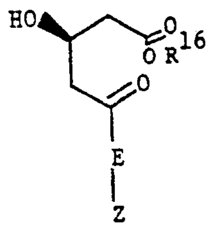

followed by treatment with alkali to produce the product wherein $R^{16}$ is an alkali metal cation, followed by acidification to produce the compound wherein $R^{16}$ is a hydrogen ion.

2. A process for the preparation of a compound of structural formula

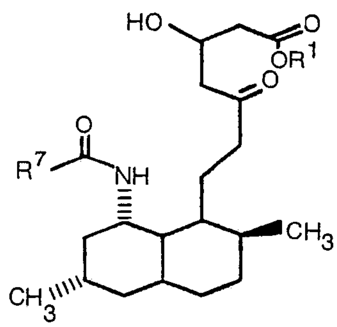

wherein $R^1$ is hydrogen, an alkali metal cation or an ammonium cation and wherein $R^7$ is 4-fluorobenzoyl, 4-tert-butylbenzoyl or 4-fluorophenylacetyl, which comprises treating as compound of structural formula:

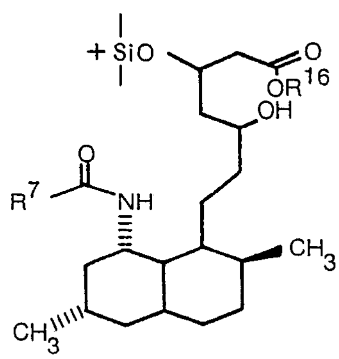

wherein $R^{16}$ is $C_{1-4}$alkyl, with an oxidizing agent to produce the compound of structural formula:

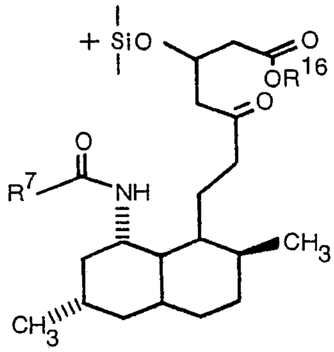

followed by desilylation to produce the compound of structural formula:

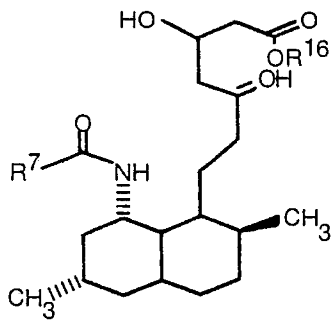

followed by treatment with alkali to produce the product wherein $R^{16}$ is an alkali metal cation, followed by acidification to produce the compound wherein $R^{16}$ is a hydrogen ion.

3. The process of Claim 1 wherein:

$R^1$ is hydrogen, an alkali metal cation or an ammonium cation;

E is $—CH=CH—$ or $—CH_2CH_2—$; and

Z is

1)

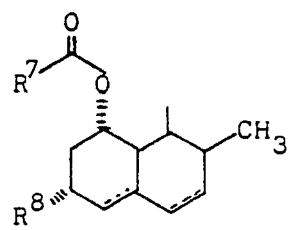

wherein

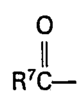

is 2(S)-methylbutyryl or 2,2-dimethylbutyryl;

2)

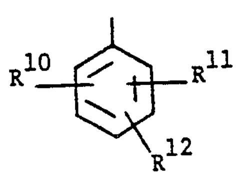

wherein $R^{10}$, $R^{11}$ and $R^{12}$ are independently

a) halogen,

b) $C_{1-4}$alkyl,

c) halo-$C_{1-4}$alkyl,

d) phenyl with 1 to 3 substituents selected from halo, $C_{1-4}$alkyl or $C_{1-4}$alkoxy,

e) $OR^{13}$, wherein $R^{13}$ is

i) phenyl,

ii) halophenyl, or

iii) phenyl-$C_{1-3}$ alkyl, either unsubstituted or substituted with one or more of halogen, $C_{1-4}$ alkoxy, $C_{1-4}$ alkyl or halo-$C_{1-4}$ alkyl; or

3) 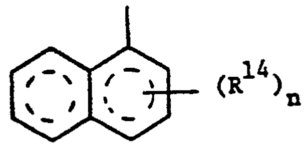

wherein n is 0, 1 or 2, and $R^{14}$ is methyl, and the ring system is naphthalene, or 5,6,7,8-tetrahydronaphthalene.

4. The process of Claim 1 for the preparation of a compound selected from:

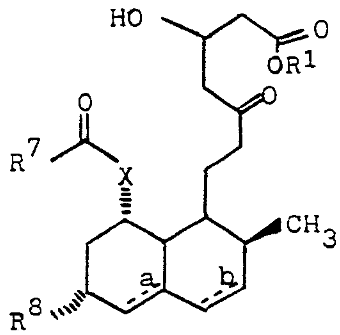

wherein $R^1$ is hydrogen, an alkali metal cation or an ammonium cation and wherein $R^7CO$—, $R^8$, X, a and b have the following meanings:

| $R^7C(=O)-$ | $R^8$ | X | a* | b |
|---|---|---|---|---|
| 2(S)-methylbutyryl | $—CH_3$ | O | single | double |
| 2(S)-methylbutyryl | $—CH_3$ | O | single | single |
| 2(R)-methylbutyryl | $—CH_3$ | O | double | double |
| 2,2-dimethylbutyryl | $—CH_3$ | O | double | double |
| 2,2-dimethylbutyryl | $—CH_3$ | O | single | double |
| 2,2-dimethylbutyryl | $—CH_3$ | O | single | single |
| acetyl | $—CH_3$ | O | double | double |
| 2(S)-methylbutyryl | H | O | double | double |
| 2(S)-methylbutyryl | H | O | single | single |
| 2,2-dimethylbutyryl | H | O | double | double |
| 2,2-dimethylbutyryl | H | O | single | single |
| 2,2-dimethylbutyryl | $—CH_3$ | NH | single | single |
| 2-methyl-2-ethylbutyryl | $—CH_3$ | NH | single | single |
| 2-methylbutyryl | $—CH_3$ | NH | single | single |
| acetyl | $—CH_3$ | NH | double | double |
| acetyl | $—CH_3$ | $NCH_3$ | single | single |
| 2,2-dimethylbutyryl | $—CH_3$ | $NCH_3$ | single | single |
| 2,2-dimethylbutyryl | $—CH_3$ | NH | double | double |

* When a = single bond, the rings are *trans*-fused.

5. The process of Claim 3 for the preparation of a compound selected from:

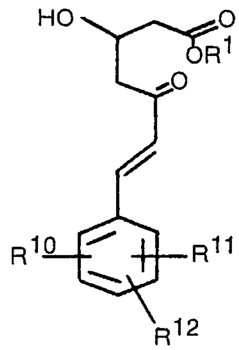

| $R^{10}$ | $R^{11}$ | $R^{12}$ |
|---|---|---|
| 6-(4-fluoro-3-methylphenyl)- | 2-methyl | 4-methyl |
| 6-(4-fluorophenyl)- | 2-chloro | 4-chloro |
| 6-(4-chlorophenyl)- | 2-chloro | 4-chloro |
| 6-(3,4-dichlorophenyl) | 2-chloro | 4-chloro |
| 6-(4-fluoro-3-methylphenyl) | 2-chloro | 4-chloro |
| 6-(3,4-dichlorophenyl) | 2-methyl | 4-methyl |
| 6-(3,5-dimethylphenyl)- | 2-chloro | 4-chloro |
| 6-(3,4-dichlorophenyl)- | 2-methyl | 5-methyl |
| 6-(4-fluorophenyl) | 2-methyl | 4-methyl |
| 6-(4-fluoro-3-methylphenyl)- | 2-methyl | 4-chloro |
| 6-(4-fluorobenzyloxy) | 2-chloro | 4-chloro |
| 6-(4-fluoro-3-methylphenyl)- | 2-chloro | 4-methyl |

6. The process of claim 3 for the preparation of a compound selected from:

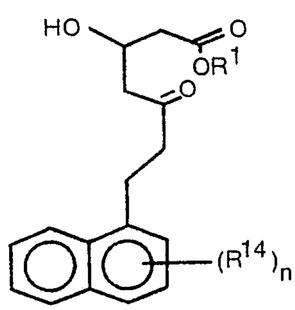

| n | $R^{14}$ | 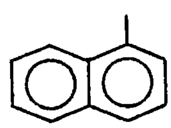 |
|---|---|---|
| 1 | 2-methyl | naphthyl |
| 0 | — | naphthyl |
| 2 | 2,6-dimethyl | naphthyl |
| 1 | 2-methyl | 5,6,7,8-tetrahydronaphthyl |

7. A process for the preparation of a compound of formula

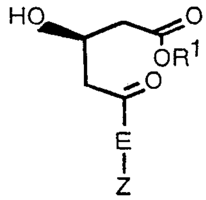

wherein:

$R^1$ is

1) hydrogen,
2) $C_{1-4}$alkyl,
3) 2,3-dihydroxypropyl,
4) alkali metal cation, or
5) ammonium of formula

$$\overset{+}{N}R^3R^4R^5R^6$$

wherein $R^3$, $R^4$, $R^5$ and $R^6$ are independently hydrogen or $C_{1-4}$alkyl or two of $R^4$, $R^4$, $R^5$ and $R^6$ are joined together to form a 5- or 6-membered heterocycle with the nitrogen to which they are attached;

E is $-CH_2CH_2$, or $-(CH_2)_3-$; and

Z is

1)

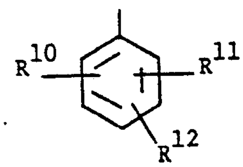

wherein $R^{10}$, $R^{11}$ and $R^{12}$ are independently

a) hydrogen,
b) halogen, such as bromo, chloro or fluoro,
c) $C_{1-4}$alkyl,
d) halo-$C_{1-4}$alkyl,
e) phenyl either unsubstituted or substituted with one or more of
   i) $C_{1-4}$alkoxy,
   ii) $C_{1-4}$alkyl,
   iii) $C_{2-8}$alkanoyloxy,
   iv) halo-$C_{1-4}$alkyl, or
   v) halo,
f) $OR^{13}$ wherein $R^{13}$ is
   i) hydrogen,
   ii) $C_{2-8}$alkanoyl,
   iii) benzoyl,
   iv) phenyl,
   v) halophenyl,
   vi) phenyl-$C_{1-3}$alkyl, either unsubstituted or substituted with one or more of halogen, $C_{1-4}$alkoxy, $C_{1-4}$alkyl or halo-$C_{1-4}$alkyl,
   vii) $C_{1-9}$alkyl,
   viii) cinnamyl,
   ix) halo-$C_{1-4}$alkyl,
   x) allyl,
   xi) $C_{3-6}$cycloalkyl-$C_{1-3}$alkyl, or
   xii) adamantyl-$C_{1-3}$alkyl;

2)

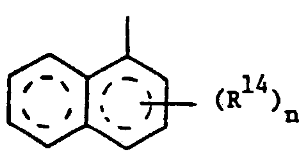

wherein n is 0—2, and $R^{14}$ is halo, or $C_{1-4}$ alkyl which comprises treating the compounds

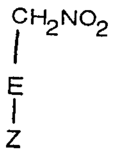

+

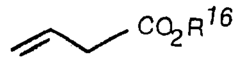

to produce the compound of structural formula:

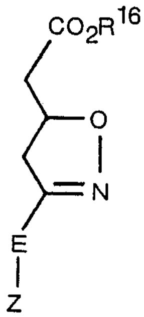

followed by catalytic reduction to produce the desired compound wherein $R^1$ is $R^{16}$; followed by treatment with alkali to produce the product wherein $R^1$ is an alkali metal cation, followed by acidification to produce the compound wherein $R^1$ is a hydrogen ion.

8. The process of Claim 7 wherein:

$R^1$ is hydrogen, an alkali metal cation or an ammonium cation;

E is $—CH_2CH_2—$; and

Z is

1) 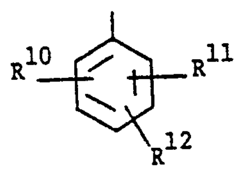

wherein $R^{10}$, $R^{11}$ and $R^{12}$ are independently

a) halogen,

b) $C_{1-4}$alkyl,

c) halo-$C_{1-4}$alkyl,

d) phenyl with 1 to 3 substituents selected from halo, $C_{1-4}$alkyl or $C_{1-4}$alkoxy,

e) $OR^{13}$, wherein $R^{13}$ is

i) phenyl,

ii) halophenyl, or

iii) phenyl-$C_{1-3}$ alkyl, either unsubstituted or substituted with one or more of halogen, $C_{1-4}$ alkoxy, $C_{1-4}$ alkyl, or halo-$C_{1-4}$ alkyl; or

2) 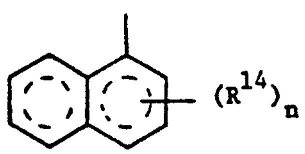

wherein n is 0, 1, or 2, and $R^{14}$ is methyl and the ring system is naphthalene or 5,6,7,8-tetrahydronaphthalene.

9. The process of Claim 8 for preparation of a compound selected from:

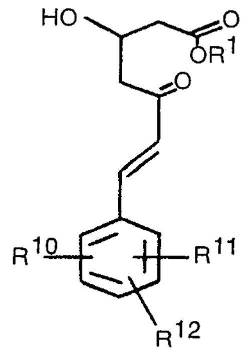

| $R^{10}$ | $R^{11}$ | $R^{12}$ |
|---|---|---|
| 6-(4-fluoro-3-methylphenyl)- | 2-methyl | 4-methyl |
| 6-(4-fluorophenyl)- | 2-chloro | 4-chloro |
| 6-(4-chlorophenyl)- | 2-chloro | 4-chloro |
| 6-(3,4-dichlorophenyl) | 2-chloro | 4-chloro |
| 6-(4-fluoro-3-methylphenyl) | 2-chloro | 4-chloro |
| 6-(3,4-dichlorophenyl) | 2-methyl | 4-methyl |
| 6-(3,5-dimethylphenyl)- | 2-chloro | 4-chloro |
| 6-(3,4-dichlorophenyl)- | 2-methyl | 5-methyl |
| 6-(4-fluorophenyl) | 2-methyl | 4-methyl |
| 6-(4-fluoro-3-methylphenyl)- | 2-methyl | 4-chloro |
| 6-(4-fluorobenzyloxy) | 2-chloro | 4-chloro |
| 6-(4-fluoro-3-methylphenyl)- | 2-chloro | 4-methyl |

10. The process of Claim 8 for the preparation of a compound selected from:

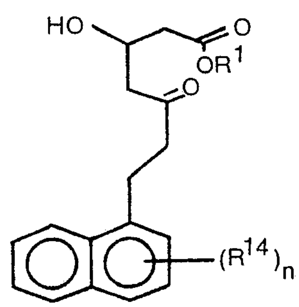

| n | $R^{14}$ | 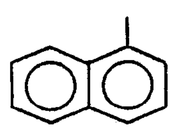 |
|---|---|---|
| 1 | 2-methyl | naphthyl |
| 0 | — | naphthyl |
| 2 | 2,6-dimethyl | naphthyl |
| 1 | 2-methyl | 5,6,7,8-tetrahydronaphthyl |

11. A process for the preparation of a compound of structural formula:

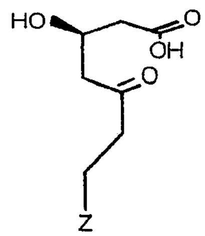

wherein Z is as defined in Claim 1, which comprises treating a compound of structural formula:

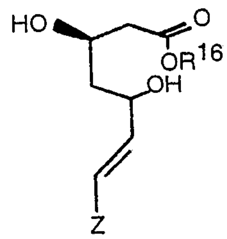

with activated manganese dioxide to produce the compound of structural formula:

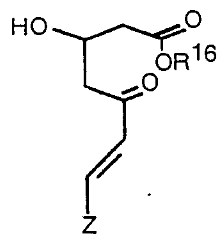

followed by treatment with tri-*n*-butyltin hydride and tetrakis (triphenylphosphine)palladium (O).

**Patentansprüche für die Vertragsstaaten: BE CH DE FE GB IT LI LU NL SE**

1. Eine Verbindung der Strukturformel:

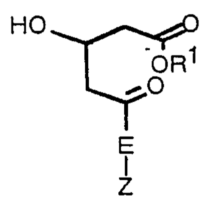

worin:

$R^1$ 1) Wasserstoff,
2) $C_{1-4}$-Alkyl,
3) 2,3-Dihydroxypropyl,
4) ein Alkalimetallkation, oder
5) ein Ammoniumkation der Formel $N^+R^3R^4R^5R^6$ ist,

wobei $R^3$, $R^4$, $R^5$ und $R^6$ unabhängig voneinander Wasserstoff oder $C_{1-4}$-Alkyl sind, oder zwei Reste von $R^3$, $R^4$, $R^5$ und $R^6$ miteinander unter Bildung eines 5- oder 6-gliedrigen Heterocyclus mit dem Stickstoff, an den sie gebunden sind, verbunden sind;

E $-CH_2CH_2$, $-CH=CH-$ oder $-(CH_2)_3-$ ist; und

Z

1)

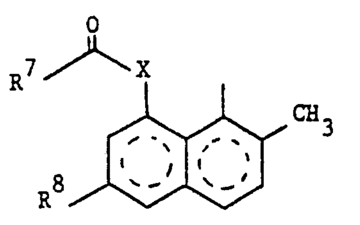

wobei die strichlierten Linien alle möglichen Oxydationszustände des bicyclischen Systems bedeuten, X —O— oder $=NR^9$ ist, wobei $R^9$ Wasserstoff oder $C_{1-3}$-Alkyl ist;

$R^7$ $C_{2-8}$-Alkyl ist; und

$R^8$ Wasserstoff oder $—CH_3$ ist;

2)

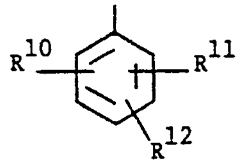

wobei $R^{10}$, $R^{11}$ und $R^{12}$ unabhängig voneinander

a) Wasserstoff,

b) Halogen, wie Brom, Chlor oder Fluor,

c) $C_{1-4}$-Alkyl,

d) Halogen-$C_{1-4}$-Alkyl,

e) Phenyl, das entweder unsubstituiert oder durch einen oder mehrere der Substituenten

i) $C_{1-4}$-Alkoxy,

ii) $C_{1-4}$-Alkyl,

iii) $C_{2-8}$-Alkanoyloxy,

iv) Halogen-$C_{1-4}$-Alkyl oder

v) Halogen, substituiert ist, oder

f) $OR^{13}$, wobei $R^{13}$

i) Wasserstoff,

ii) $C_{2-8}$-Alkanoyl,

iii) Benzoyl,

iv) Phenyl,

v) Halogenphenyl,

vi) Phenyl-$C_{1-3}$-alkyl, das entweder unsubstituiert oder durch einen oder mehrere Halogen-$C_{1-4}$-Alkoxy-, $C_{1-4}$-Alkyl- oder Halogen-$C_{1-4}$-Alkylreste substituiert ist,

vii) $C_{1-9}$-Alkyl,

viii) Zinnamyl,

ix) Halogen-$C_{1-4}$-alkyl,

x) Allyl,

xi) $C_{3-6}$-Cycloalkyl-$C_{1-3}$-alkyl, oder

xii) Adamantyl-$C_{1-3}$-alkyl ist; sind,

3)

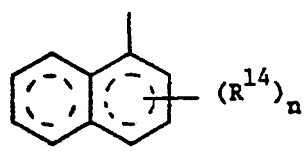

wobei n 0—2 ist, und $R^{14}$ Halogen oder $C_{1-4}$-Alkyl ist; und

4)

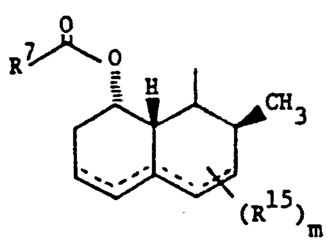

wobei die strichlierten Linien mögliche Doppelbindungen bedeuten und 0, 1 oder 2 Doppelbindungen vorliegen, ist;

m 1, 2 oder 3 bedeutet; und

$R^{15}$ 1) Methyl,

2) Hydroxy,

3) $C_{1-4}$-Alkoxy,

4) Oxo oder

5) Halogen ist.

2. Eine Verbindung der Strukturformel

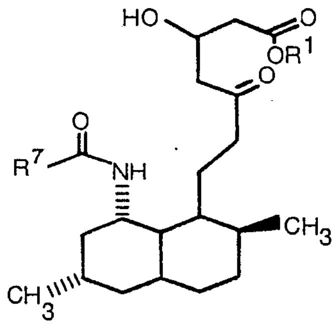

worin $R^1$ Wasserstoff, ein Alkalimetallkation oder ein Ammoniumkation ist, und worin $R^7$ 4-Fluorbenzoyl, 4-tert.-Butylbenzoyl oder 4-Fluorphenylacetyl ist.

3. Die Verbindung von Anspruch 1, worin

$R^1$ Wasserstoff, ein Alkalilmetallkation oder ein Ammoniumkation ist;

E —CH=CH— oder —$CH_2CH_2$— ist; und

Z

1)

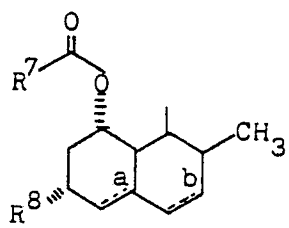

wobei

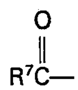

2(S)-Methylbutyryl oder 2,2-Dimethylbutyryl ist;

2)

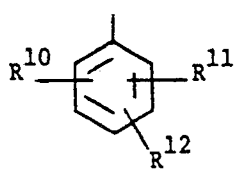

wobei $R^{10}$, $R^{11}$ und $R^{12}$ unabhängig voneinander

a) Halogen,

b) $C_{1-4}$-Alkyl,

c) Halogen-$C_{1-4}$-alkyl,

d) Phenyl mit 1 bis 3 Substituenten, ausgewählt aus Halogen, $C_{1-4}$-Alkyl oder $C_{1-4}$-Alkoxy, oder

e) $OR^{13}$, wobei $R^{13}$

i) Phenyl,

ii) Halogenphenyl oder

iii) Phenyl-$C_{1-3}$-alkyl ist, das entweder unsubstituiert oder durch einen oder mehrere Halogen-, $C_{1-4}$-Alkoxy-, $C_{1-4}$-Alkyl- oder Halogen-$C_{1-4}$-alkylreste substituierte ist, darstellen; oder

3)

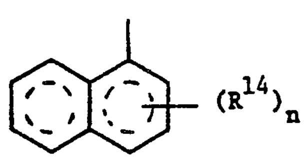

wobei n 0, 1 oder 2 ist, und $R^{14}$ Methyl ist, und das Ringsystem Naphthyl oder 5,6,7,8-Tetrahydronaphthyl ist, bedeutet.

4. Die Verbindung von Anspruch 1, ausgewählt aus:

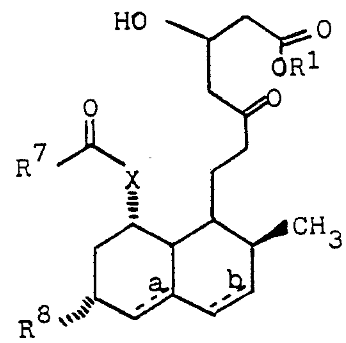

worin $R^1$ Wasserstoff, ein Alkalimetallkation oder ein Ammoniumkation ist, und worin $R^7CO$—, $R^8$, X, a und b die folgenden Bedeutungen haben:

| $R^7C(=O)-$ | $R^8$ | X | a* | b |
|---|---|---|---|---|
| 2(S)-Methylbutyryl | $-CH_3$ | O | Einfachbindung | Doppelbindung |
| 2(S)-Methylbutyryl | $-CH_3$ | O | Einfachbindung | Einfachbindung |
| 2(R)-Methylbutyryl | $-CH_3$ | O | Doppelbindung | Doppelbindung |
| 2,2-Dimethylbutyryl | $-CH_3$ | O | Doppelbindung | Doppelbindung |
| 2,2-Dimethylbutyryl | $-CH_3$ | O | Einfachbindung | Doppelbindung |
| 2,2-Dimethylbutyryl | $-CH_3$ | O | Einfachbindung | Einfachbindung |
| Acetyl | $-CH_3$ | O | Doppelbindung | Doppelbindung |
| 2(S)-Methylbutyryl | H | O | Doppelbindung | Doppelbindung |
| 2(S)-Methylbutyryl | H | O | Einfachbindung | Einfachbindung |
| 2,2-Dimethylbutyryl | H | O | Doppelbindung | Doppelbindung |
| 2,2-Dimethylbutyryl | H | O | Einfachbindung | Einfachbindung |
| 2,2-Dimethylbutyryl | $-CH_3$ | NH | Einfachbindung | Einfachbindung |
| 2-Methyl-2-ethylbutyryl | $-CH_3$ | NH | Einfachbindung | Einfachbindung |
| 2-Methylbutyryl | $-CH_3$ | NH | Einfachbindung | Einfachbindung |
| Acetyl | $-CH_3$ | NH | Doppelbindung | Doppelbindung |
| Acetyl | $-CH_3$ | $NCH_3$ | Einfachbindung | Einfachbindung |
| 2,2-Dimethylbutyryl | $-CH_3$ | $NCH_3$ | Einfachbindung | Einfachbindung |
| 2,2-Dimethylbutyryl | $-CH_3$ | NH | Doppelbindung | Doppelbindung |

* Falls a eine Einfachbindung ist, sind die Ringe trans-kondensiert.

5. Die Verbindung von Anspruch 3, ausgewählt aus:

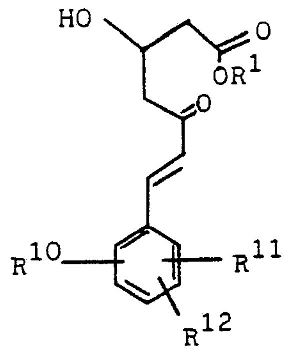

| $R^{10}$ | $R^{11}$ | $R^{12}$ |
|---|---|---|
| 6-(4-Fluor-3-methylphenyl)- | 2-Methyl | 4-Methyl |
| 6-(4-Fluorphenyl)- | 2-Chlor | 4-Chlor |
| 6-(4-Chlorphenyl)- | 2-Chlor | 4-Chlor |
| 6-(3,4-Dichlorphenyl) | 2-Chlor | 4-Chlor |
| 6-(4-Fluor-3-methylphenyl) | 2-Chlor | 4-Chlor |
| 6-(3,4-Dichlorphenyl) | 2-Methyl | 4-Methyl |
| 6-(3,5-Dimethylphenyl)- | 2-Chlor | 4-Chlor |
| 6-(3,4-Dichlorphenyl)- | 2-Methyl | 5-Methyl |
| 6-(4-Fluorphenyl) | 2-Methyl | 4-Methyl |
| 6-(4-Fluor-3-methylphenyl)- | 2-Methyl | 4-Chlor |
| 6-(4-Fluorbenzyloxy) | 2-Chlor | 4-Chlor |
| 6-(4-Fluor-3-methylphenyl)- | 2-Chlor | 4-Methyl |

6. Die Verbindung von Anspruch 3, ausgewählt aus:

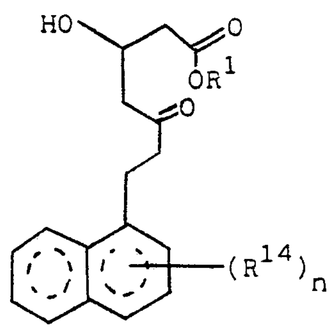

| n | $R^{14}$ | 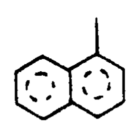 |
|---|---|---|
| 1 | 2-Methyl | Naphthyl |
| 0 | - | Naphthyl |
| 2 | 2,6-Dimethyl | Naphthyl |
| 1 | 2-Methyl | 5,6,7,8-Tetrahydro-naphthyl |

7. Eine antihypercholesterinämische, pharmazeutische Zusammensetzung, enthaltend einen pharmazeutischen Träger und eine antihypercholesterinämisch wirksame Menge einer Verbindung wie in Anspruch 1 oder 2 beansprucht.

8. Die Formulierung von Anspruch 7, wobei die antihypercholesterinämische Verbindung eine in Anspruch 3 beanspruchte Verbindung ist.

9. Die Formulierung von Anspruch 8, wobei die antihypercholesterinämische Verbindung eine in den Ansprüchen 4, 5 oder 6 beanspruchte Verbindung ist.

10. Ein Verfahren zur Herstellung einer Verbindung der Strukturformel:

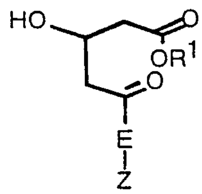

worin $R^1$, E und Z die in Anspruch 1 angegebenen Bedeutungen von $R^1$, E und Z 1), 2) und 3) haben, welches das Behandeln einer Verbindung der Strukturformel

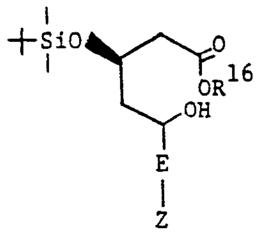

worin $R^{16}$ $C_{1-4}$-Alkyl ist, mit einem Oxydationsmittel unter Bildung der Verbindung der Strukturformel:

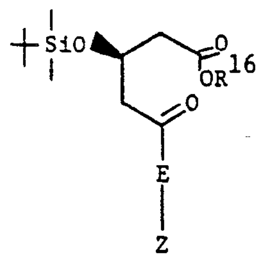

gefolgt von der Entsilylierung unter Bildung der Verbindung der Strukturformel:

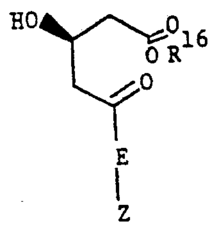

gefolgt von der Behandlung mit Alkali unter Bildung des Produktes, worin $R^{16}$ ein Alkalimetallkation ist, gefolgt von der Ansäuerung unter Bildung der Verbindung, worin $R^{16}$ ein Wasserstoffion ist, umfaßt.

**Patentansprüche für den Vertragsstaat: AT**

1. Eine Verfahren zur Herstellung einer Verbindung der Strukturformel:

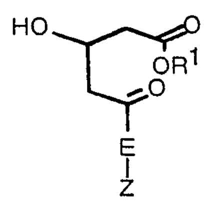

worin:

$R^1$ 1) Wasserstoff,

2) $C_{1-4}$-Alkyl,

3) 2,3-Dihydroxypropyl,
4) ein Alkalimetallkation, oder
5) ein Ammoniumkation der Formel $N^+R^3R^4R^5R^6$ ist,

wobei $R^3$, $R^4$, $R^5$ und $R^6$ unabhängig voneinander Wasserstoff oder $C_{1-4}$-Alkyl sind, oder zwei Reste von $R^3$, $R^4$, $R^5$ und $R^6$ miteinander unter Bildung eines 5- oder 6-gliedrigen Heterocyclus mit dem Stickstoff, an den sie gebunden sind, verbunden sind;

E $-CH_2CH_2$, $-CH=CH-$ oder $-(CH_2)_3-$ ist; und

Z

1) 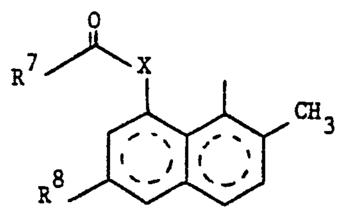

wobei die strichlierten Linien alle möglichen Oxydationszustände des bicyclischen Systems bedeuten, X $-O-$ oder $=NR^9$ ist, wobei $R^9$ Wasserstoff oder $C_{1-3}$-Alkyl ist;

$R^7$ $C_{2-8}$-Alkyl ist; und

$R^8$ Wasserstoff oder $-CH_3$ ist;

2) 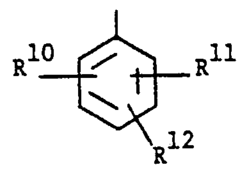

wobei $R^{10}$, $R^{11}$ und $R^{12}$ unabhängig voneinander

a) Wasserstoff,
b) Halogen, wie Brom, Chlor oder Fluor,
c) $C_{1-4}$-Alkyl,
d) Halogen-$C_{1-4}$-Alkyl,
e) Phenyl, das entweder unsubstituiert oder durch einen oder mehrere der Substituenten
  i) $C_{1-4}$-Alkoxy,
  ii) $C_{1-4}$-Alkyl,
  iii) $C_{2-8}$-Alkanoyloxy,
  iv) Halogen-$C_{1-4}$-Alkyl oder
  v) Halogen, substituiert ist, oder
f) $OR^{13}$, wobei $R^{13}$
  i) Wasserstoff,
  ii) $C_{2-8}$-Alkanoyl,
  iii) Benzoyl,
  iv) Phenyl,
  v) Halogenphenyl,
  vi) Phenyl-$C_{1-3}$-alkyl, das entweder unsubstituiert oder durch einen oder mehrere Halogen-$C_{1-4}$-Alkoxy-, $C_{1-4}$-Alkyl- oder Halogen-$C_{1-4}$-Alkylreste substituiert ist,
  vii) $C_{1-9}$-Alkyl,
  viii) Zinnamyl,
  ix) Halogen-$C_{1-4}$-alkyl,
  x) Allyl,
  xi) $C_{3-6}$-Cycloalkyl-$C_{1-3}$-alkyl, oder
  xii) Adamantyl-$C_{1-3}$-alkyl ist, sind; oder

3) 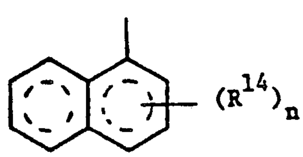

wobei n 0—2 ist, und $R^{14}$ Halogen oder $C_{1-4}$-Alkyl ist; bedeutet;

welches das Behandeln einer Verbindung der Strukturformel

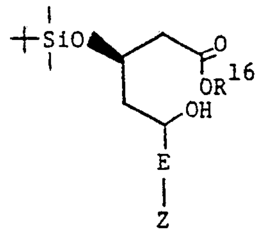

worin $R^{16}$ $C_{1-4}$-Alkyl ist, mit einem Oxydationsmittel unter Bilding der Verbindung der Strukturformel:

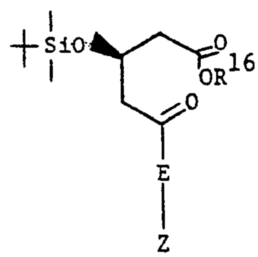

gefolgt von der Entsilylierung unter Bilding der Verbindung der Strukturformel:

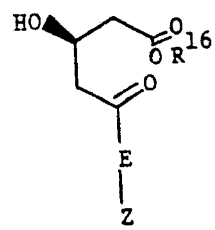

gefolgt von der Behandlung mit Alkali unter Bildung des Produktes, worin $R^{16}$ ein Alkalimetallkation ist, gefolgt von der Ansäuerung unter Bilding der Verbindung, worin $R^{16}$ ein Wasserstoffion ist, umfaßt.

2. Ein Verfahren zur Herstellung einer Verbindung der Strukturformel

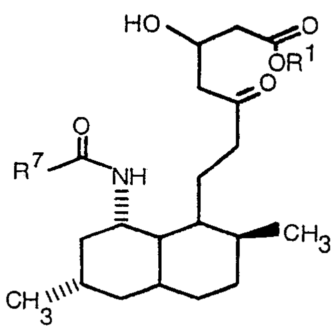

worin $R^1$ Wasserstoff, ein Alkalimetallkation oder ein Ammoniumkation ist, und worin $R^7$ 4-Fluorbenzoyl, 4-tert.-Butylbenzoyl oder 4-Fluorphenylacetyl ist,
welches das Behandeln einer Verbindung der Strukturformel

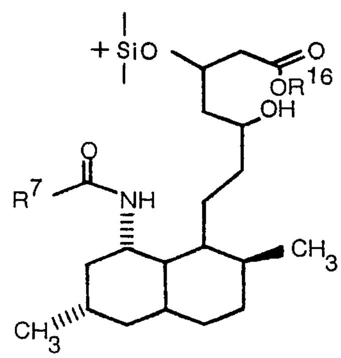

worin $R^{16}$ $C_{1-4}$-Alkyl ist, mit einem Oxydationsmittel unter Bildung der Verbindung der Strukturformel:

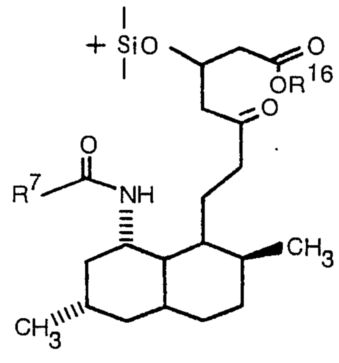

gefolgt von der Entsilylierung unter Bilding der Verbindung der Strukturformel:

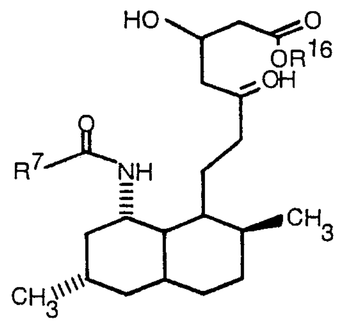

gefolgt von der Behandlung mit Alkali unter Bilding des Produktes, worin $R^{16}$ ein Alkalimetallkation ist, gefolgt von der Ansäuerung unter Bilding der Verbindung, worin $R^{16}$ ein Wasserstoffion ist, umfaßt.

3. Das Verfahren von Anspruch 1, worin
$R^1$ Wasserstoff, ein Alkalilmetallkation oder ein Ammoniumkation ist;
E $-CH{=}CH-$ oder $-CH_2CH_2-$ ist; und
Z

1)

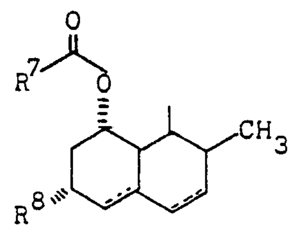

wobei

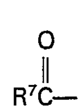

2(S)-Methylbutyryl oder 2,2-Dimethylbutyryl ist;

2)

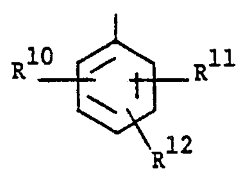

wobei $R^{10}$, $R^{11}$ und $R^{12}$ unabhängig voneinander
a) Halogen,
b) $C_{1-4}$-Alkyl,
c) Halogen-$C_{1-4}$-alkyl,
d) Phenyl mit 1 bis 3 Substituenten, ausgewählt aus Halogen, $C_{1-4}$-Alkyl oder $C_{1-4}$-Alkoxy, oder
e) $OR^{13}$, wobei $R^{13}$
i) Phenyl,

ii) Halogenphenyl oder

iii) Phenyl-$C_{1-3}$-alkyl ist, das entweder unsubstituiert oder durch einen oder mehrere Halogen-, $C_{1-4}$-Alkoxy-, $C_{1-4}$-Alkyl- oder Halogen-$C_{1-4}$-alkylreste substituierte ist, darstellen; oder

3) 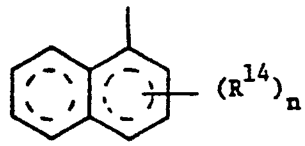

wobei n 0, 1 oder 2 ist, und $R^{14}$ Methyl ist, und das Ringsystem Naphthyl oder 5,6,7,8-Tetrahydronaphthyl ist, bedeutet.

4. Das Verfahren von Anspruch 1 zur Herstellung einer Verbindung, ausgewählt aus:

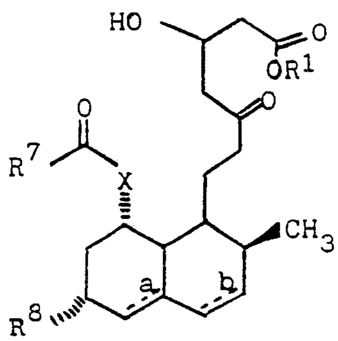

worin $R^1$ Wasserstoff, ein Alkalimetallkation oder ein Ammoniumkation ist, und worin $R^7CO$—, $R^8$, X, a und b die folgenden Bedeutungen haben:

| $R^7\overset{O}{\overset{\|}{C}}$- | $R^8$ | X | a* | b |
|---|---|---|---|---|
| 2(S)-Methylbutyryl | $-CH_3$ | O | Einfachbindung | Doppelbindung |
| 2(S)-Methylbutyryl | $-CH_3$ | O | Einfachbindung | Einfachbindung |
| 2(R)-Methylbutyryl | $-CH_3$ | O | Doppelbindung | Doppelbindung |
| 2,2-Dimethylbutyryl | $-CH_3$ | O | Doppelbindung | Doppelbindung |
| 2,2-Dimethylbutyryl | $-CH_3$ | O | Einfachbindung | Doppelbindung |
| 2,2-Dimethylbutyryl | $-CH_3$ | O | Einfachbindung | Einfachbindung |
| Acetyl | $-CH_3$ | O | Doppelbindung | Doppelbindung |
| 2(S)-Methylbutyryl | H | O | Doppelbindung | Doppelbindung |
| 2(S)-Methylbutyryl | H | O | Einfachbindung | Einfachbindung |
| 2,2-Dimethylbutyryl | H | O | Doppelbindung | Doppelbindung |
| 2,2-Dimethylbutyryl | H | O | Einfachbindung | Einfachbindung |
| 2,2-Dimethylbutyryl | $-CH_3$ | NH | Einfachbindung | Einfachbindung |
| 2-Methyl-2-ethylbutyryl | $-CH_3$ | NH | Einfachbindung | Einfachbindung |
| 2-Methylbutyryl | $-CH_3$ | NH | Einfachbindung | Einfachbindung |
| Acetyl | $-CH_3$ | NH | Doppelbindung | Doppelbindung |
| Acetyl | $-CH_3$ | $NCH_3$ | Einfachbindung | Einfachbindung |
| 2,2-Dimethylbutyryl | $-CH_3$ | $NCH_3$ | Einfachbindung | Einfachbindung |
| 2,2-Dimethylbutyryl | $-CH_3$ | NH | Doppelbindung | Doppelbindung |

* Falls a eine Einfachbindung ist, sind die Ringe trans-kondensiert.

5. Das Verfahren von Anspruch 3 zur Herstellung einer Verbindung, ausgewählt aus:

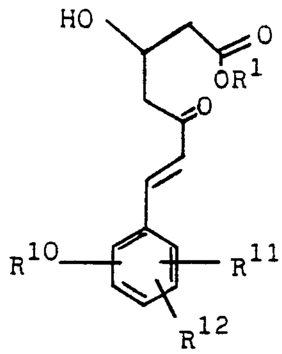

| $R^{10}$ | $R^{11}$ | $R^{12}$ |
|---|---|---|
| 6-(4-Fluor-3-methylphenyl)- | 2-Methyl | 4-Methyl |
| 6-(4-Fluorphenyl)- | 2-Chlor | 4-Chlor |
| 6-(4-Chlorphenyl)- | 2-Chlor | 4-Chlor |
| 6-(3,4-Dichlorphenyl) | 2-Chlor | 4-Chlor |
| 6-(4-Fluor-3-methylphenyl) | 2-Chlor | 4-Chlor |
| 6-(3,4-Dichlorphenyl) | 2-Methyl | 4-Methyl |
| 6-(3,5-Dimethylphenyl)- | 2-Chlor | 4-Chlor |
| 6-(3,4-Dichlorphenyl)- | 2-Methyl | 5-Methyl |
| 6-(4-Fluorphenyl) | 2-Methyl | 4-Methyl |
| 6-(4-Fluor-3-methylphenyl)- | 2-Methyl | 4-Chlor |
| 6-(4-Fluorbenzyloxy) | 2-Chlor | 4-Chlor |
| 6-(4-Fluor-3-methylphenyl)- | 2-Chlor | 4-Methyl |

6. Das Verfahren von Anspruch 3 zur Herstellung einer Verbindung, ausgewählt aus:

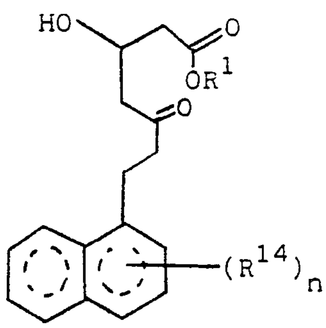

| n | $R^{14}$ | 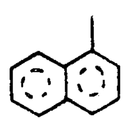 |
|---|---|---|
| 1 | 2-Methyl | Naphthyl |
| 0 | - | Naphthyl |
| 2 | 2,6-Dimethyl | Naphthyl |
| 1 | 2-Methyl | 5,6,7,8-Tetrahydro-naphthyl |

7. Ein Verfahren zur Herstellung einer Verbindung der Formel

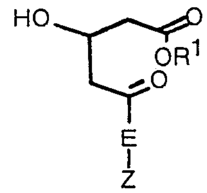

worin:

$R^1$ 1) Wasserstoff,
2) $C_{1-4}$-Alkyl,
3) 2,3-Dihydroxypropyl,
4) ein Alkalimetallkation, oder
5) ein Ammoniumkation der Formel $N^+R^3R^4R^5R^6$ ist,

wobei $R^3$, $R^4$, $R^5$ und $R^6$ unabhängig voneinander Wasserstoff oder $C_{1-4}$-Alkyl sind, oder zwei Reste von $R^3$, $R^4$, $R^5$ und $R^6$ miteinander unter Bildung eines 5- oder 6-gliedrigen Heterocyclus mit dem Stickstoff, an den sie gebunden sind, verbunden sind;

E $—CH_2CH_2$ oder $—(CH_2)_3—$ ist; und

Z

1)

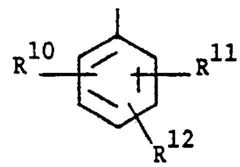

wobei $R^{10}$, $R^{11}$ und $R^{12}$ unabhängig voneinander

a) Wasserstoff,
b) Halogen, wie Brom, Chlor oder Fluor,
c) $C_{1-4}$-Alkyl,
d) Halogen-$C_{1-4}$-Alkyl,
e) Phenyl, das entweder unsubstituiert oder durch einen oder mehrere der Substituenten
   i) $C_{1-4}$-Alkoxy,
   ii) $C_{1-4}$-Alkyl,
   iii) $C_{2-8}$-Alkanoyloxy,
   iv) Halogen-$C_{1-4}$-Alkyl oder
   v) Halogen, substituiert ist, oder
f) $OR^{13}$, wobei $R^{13}$
   i) Wasserstoff,
   ii) $C_{2-8}$-Alkanoyl,
   iii) Benzoyl,
   iv) Phenyl,
   v) Halogenphenyl,
   vi) Phenyl-$C_{1-3}$-alkyl, das entweder unsubstituiert oder durch einen oder mehrere Halogen-$C_{1-4}$-Alkoxy-, $C_{1-4}$-Alkyl- oder Halogen-$C_{1-4}$-Alkylreste substituiert ist,
   vii) $C_{1-9}$-Alkyl,
   viii) Zinnamyl,
   ix) Halogen-$C_{1-4}$-alkyl,
   x) Allyl,
   xi) $C_{3-6}$-Cycloalkyl-$C_{1-3}$-alkyl, oder
   xii) Adamantyl-$C_{1-3}$-alkyl ist, sind;

2)

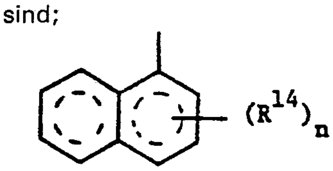

wobei n 0—2 ist, und $R^{14}$ Halogen oder $C_{1-4}$-Alkyl ist; bedeutet; welches das Umsetzen der Verbindungen

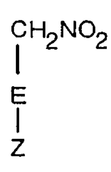

+

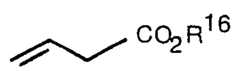

unter Bildung der Verbindung der Strukturformel:

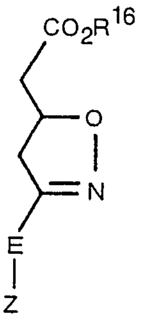

gefolgt von der katalytischen Reduktion unter Bildung der gewünschten Verbindung, worin $R^1$ $R^{16}$ ist; gefolgt von der Behandlung mit Alkali unter Bilding des Produktes, worin $R^1$ ein Alkalimetallkation ist, gefolgt von der Ansäuerung unter Bilding der Verbindung, worin $R^1$ ein Wasserstoffion ist, umfaßt.

8. Das Verfahren von Anspruch 7, worin:
$R^1$ Wasserstoff, ein Alkalimetallkation oder ein Ammoniumkation ist;
E $-CH_2CH_2-$ ist; und
Z

1) 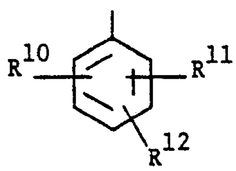

wobei $R^{10}$, $R^{11}$ und $R^{12}$ unabhängig voneinander
a) Halogen,
b) $C_{1-4}$-alkyl,
c) Halogen-$C_{1-4}$-alkyl,
d) Phenyl mit 1 bis 3 Substituenten, ausgewählt aus Halogen, $C_{1-4}$-Alkyl oder $C_{1-4}$-Alkoxy, oder
e) $OR^{13}$, wobei $R^{13}$
i) Phenyl,
ii) Halogenphenyl oder
iii) Phenyl-$C_{1-3}$-alkyl ist, das entweder unsubstituiert oder durch einen oder mehrere Halogen-, $C_{1-4}$-Alkoxy-, $C_{1-4}$-Alkyl- oder Halogen-$C_{1-4}$-alkylreste substituiert ist, darstellen; oder

2) 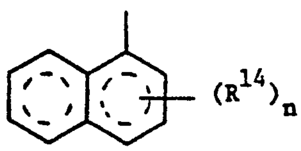

wobei n 0, 1 oder 2 ist, und $R^{14}$ Methyl ist, und das Ringsystem Naphthyl oder 5,6,7,8-Tetrahydronaphthyl ist, bedeutet.

9. Das Verfahren von Anspruch 8 zur Herstellung einer Verbindung, ausgewählt aus:

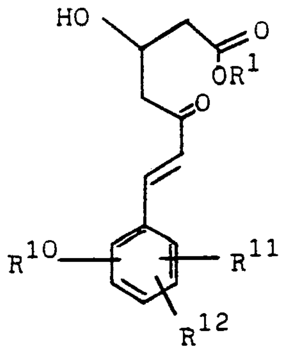

| $R^{10}$ | $R^{11}$ | $R^{12}$ |
|---|---|---|
| 6-(4-Fluor-3-methylphenyl)- | 2-Methyl | 4-Methyl |
| 6-(4-Fluorphenyl)- | 2-Chlor | 4-Chlor |
| 6-(4-Chlorphenyl)- | 2-Chlor | 4-Chlor |
| 6-(3,4-Dichlorphenyl) | 2-Chlor | 4-Chlor |
| 6-(4-Fluor-3-methylphenyl) | 2-Chlor | 4-Chlor |
| 6-(3,4-Dichlorphenyl) | 2-Methyl | 4-Methyl |
| 6-(3,5-Dimethylphenyl)- | 2-Chlor | 4-Chlor |
| 6-(3,4-Dichlorphenyl)- | 2-Methyl | 5-Methyl |
| 6-(4-Fluorphenyl) | 2-Methyl | 4-Methyl |
| 6-(4-Fluor-3-methylphenyl)- | 2-Methyl | 4-Chlor |
| 6-(4-Fluorbenzyloxy) | 2-Chlor | 4-Chlor |
| 6-(4-Fluor-3-methylphenyl)- | 2-Chlor | 4-Methyl |

10. Das Verfahren von Anspruch 8 zur Herstellung einer Verbindung, ausgewählt aus:

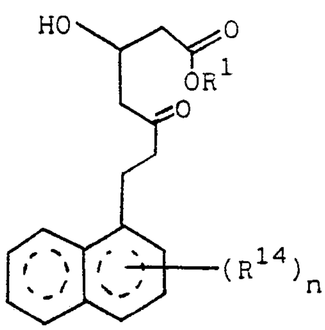

| n | $R^{14}$ | 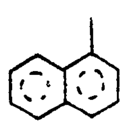 |
|---|---|---|
| 1 | 2-Methyl | Naphthyl |
| 0 | - | Naphthyl |
| 2 | 2,6-Dimethyl | Naphthyl |
| 1 | 2-Methyl | 5,6,7,8-Tetrahydro-naphthyl |

11. Ein Verfahren zur Herstellung einer Verbindung der Strukturformel:

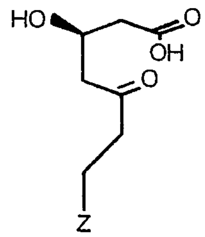

worin Z wie in Anspruch 1 definiert ist,
welches das Behandeln einer Verbindung der Strukturformel:

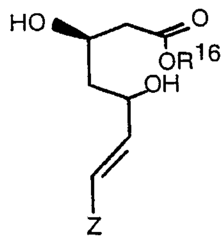

mit aktiviertem Mangandioxid unter Bildung der Verbindung der Strukturformel:

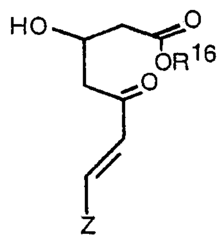

gefolgt von der Behandlung mit Tri-n-butylzinnhydrid und Tetrakis(triphenylphosphin)palladium (O), umfaßt.

**Revendications pour les Etats contractants: BE CH DE FR GB IT LI LU NL SE**

1. Un composé répondant à la formule développée:

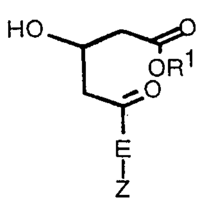

dans laquelle:

$R^1$ est

1) un hydrogène,

2) un alkyle en $C_{1-4}$,

3) un 2,3-dihydroxypropyle,

4) un cation de métal alcalin ou

5) un ammonium de formule $N^+R^3R^4R^5R^6$ dans laquelle $R^3$, $R^4$, $R^5$ et $R^6$ sont indépendamment un hydrogène ou un alkyle en $C_{1-4}$ ou deux de $R^3$, $R^4$, $R^5$ et $R^6$ sont réunis pour former un hétérocycle à 5 ou 6 chaînons avec l'azote auquel ils sont fixés;

E est $-CH_2CH_2-$, $-CH{=}CH-$ ou $-(CH_2)_3-$; et

Z est

1)

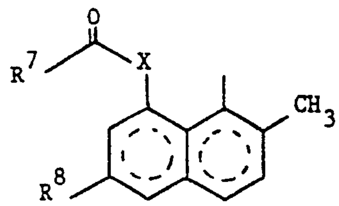

où les pointillés représentent tous les états d'oxydation possibles du système bicyclique;

X est —O— ou >$NR^9$ où $R^9$ est un hydrogène ou un alkyle en $C_{1-3}$;

$R^7$ est un alkyle en $C_{2-8}$; et

$R^8$ est un hydrogène ou —$CH_3$;

2)

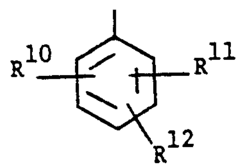

où $R^{10}$, $R^{11}$ et $R^{12}$ sont indépendamment

a) un hydrogène,

b) un halogène, tel que bromo, chloro ou fluoro,

c) un alkyle en $C_{1-4}$,

d) un halogénoalkyle en $C_{1-4}$,

e) un phényle soit non substitué soit substitué par un ou plusieurs de

i) alcoxy en $C_{1-4}$,

ii) alkyle en $C_{1-4}$,

iii) alcanoyloxy en $C_{2-8}$,

iv) halogénoalkyle en $C_{1-4}$ ou

v) halogéno, tel que bromo, chloro ou fluoro,

f) $OR^{13}$ où $R^{13}$ est

i) un hydrogène,

ii) un alcanoyle en $C_{2-8}$,

iii) un benzoyle,

iv) un phényle,

v) un halogénophényle,

vi) un phényl-alkyle en $C_{1-3}$ soit non substitué soit substitué par un ou plusieurs halogènes, alcoxy en $C_{1-4}$, alkyles en $C_{1-4}$ ou halogénoalkyles en $C_{1-4}$,

vii) un alkyle en $C_{1-9}$,

viii) un cinnamyle,

ix) un halogénoalkyle en $C_{1-4}$,

x) un allyle,

xi) un cycloalkyl($C_{3-6}$)-alkyle en $C_{1-3}$ ou

xii) un adamantyl-alkyle en $C_{1-3}$,

3)

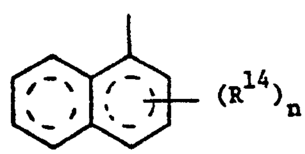

où n est 0—2 et $R^{14}$ est un halogéno ou un alkyle en $C_{1-4}$; et

4)

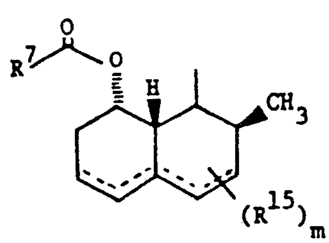

où les pointillés représentent les doubles liaisons possibles, 0, 1 ou 2 doubles liaisons pouvant exister; m représente 1, 2 ou 3; et

$R^{15}$ est

1) un méthyle,
2) un hydroxy,
3) un alcoxy en $C_{1-4}$,
4) un oxo ou
5) un halogéno.

2. Un composé répondant à la formule développée:

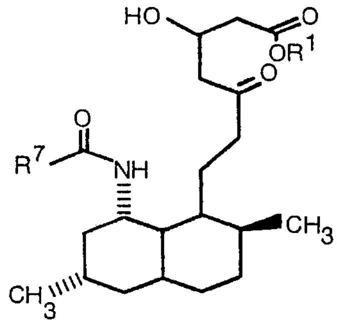

dans laquelle $R^1$ est un hydrogène, un cation de métal alcalin ou un cation ammonium et $R^7$ est un 4-fluorobenzoyle, un 4-tert-butyl-benzoyle ou un 4-fluorophénylacétyle.

3. Le composé de la revendication 1 où:
$R^1$ est un hydrogène, un cation de métal alcalin ou un cation ammonium;
E est $-CH{=}CH-$ ou $-CH_2CH_2-$; et
Z est

1)

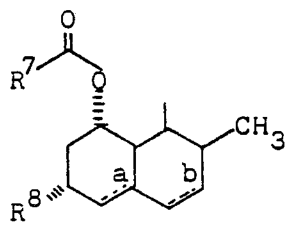

dans laquelle

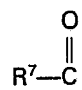

est un 2(S)-méthylbutyryle ou un 2-2-diméthylbutyryle;

2)

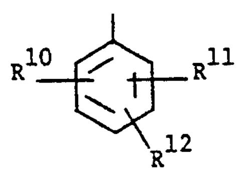

où $R^{10}$, $R^{11}$ et $R^{12}$ sont indépendamment:
a) un halogène,
b) un alkyle en $C_{1-4}$,
c) un halogénoalkyle en $C_{1-4}$,
d) un phényle ayant 1 à 3 substituants choisis parmi halogéno, alkyle en $C_{1-4}$ ou alcoxy en $C_{1-4}$,
e) $OR^{13}$, où $R^{13}$ est
i) un phényle,
ii) un halogénophényle, ou
iii) un phényl-alkyle en $C_{1-3}$ soit non substitué soit substitué par un ou plusieurs halogènes, alcoxy en $C_{1-4}$, alkyles en $C_{1-4}$ ou halogénoalkyles en $C_{1-4}$; ou

3)

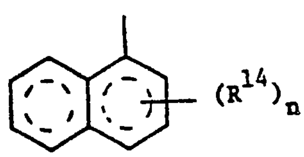

où n est 0, 1 ou 2 et $R^{14}$ est un méthyle et le système cyclique est un naphtyle ou un 5,6,7,8-tétrahydronaphtyle.

4. Le composé de la revendication 1 choisi parmi:

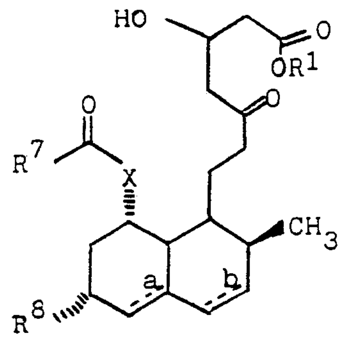

où $R^1$ est un hydrogène, un cation de métal alcalin ou un cation ammonium et où $R^7CO—$, $R^8$, X, a et b ont les significations suivantes:

| $R^7-\overset{O}{\overset{\parallel}{C}}-$ | $R^8$ | x | a* | b |
|---|---|---|---|---|
| 2(S)-méthylbutyryle | $-CH_3$ | O | simple | double |
| 2(S)-méthylbutyryle | $-CH_3$ | O | simple | simple |
| 2(R)-méthylbutyryle | $-CH_3$ | O | double | double |
| 2,2-diméthylbutyryle | $-CH_3$ | O | double | double |
| 2,2-diméthylbutyryle | $-CH_3$ | O | simple | double |
| 2,2-diméthylbutyryle | $-CH_3$ | O | simple | simple |
| acétyle | $-CH_3$ | O | double | double |
| 2(S)-méthylbutyryle | H | O | double | double |
| 2(S)-méthylbutyryle | H | O | simple | simple |
| 2,2-diméthylbutyryle | H | O | double | double |
| 2,2-diméthylbutyryle | H | O | simple | simple |
| 2,2-diméthylbutyryle | $-CH_3$ | NH | simple | simple |
| 2-méthyl-2-éthylbutyryle | $-CH_3$ | NH | simple | simple |
| 2-méthylbutyryle | $-CH_3$ | NH | simple | simple |
| acétyle | $-CH_3$ | NH | double | double |
| acétyle | $-CH_3$ | $NCH_3$ | simple | simple |
| 2,2-diméthylbutyryle | $-CH_3$ | $NCH_3$ | simple | simple |
| 2,2-diméthylbutyryle | $-CH_3$ | NH | double | double |

* lorsque a = simple liaison, les cycles sont condensés en trans.

5. Le composé de la revendication 3 choisi parmi

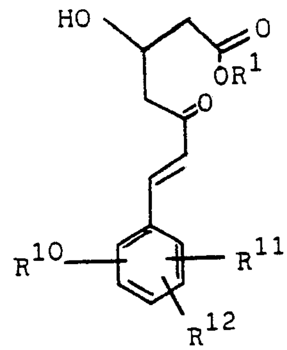

| $R^{10}$ | $R^{11}$ | $R^{12}$ |
|---|---|---|
| 6-(4-fluoro-3-méthylphényl)- | 2-méthyl | 4-méthyl |
| 6-(4-fluorophényl)- | 2-chloro | 4-chloro |
| 6-(4-chlorophényl)- | 2-chloro | 4-chloro |
| 6-(3,4-dichlorophényl)- | 2-chloro | 4-chloro |
| 6-(4-fluoro-3-méthylphényl)- | 2-chloro | 4-chloro |
| 6-(3,4-dichlorophényl)- | 2-méthyl | 4-méthyl |
| 6-(3,5-diméthylphényl)- | 2-chloro | 4-chloro |
| 6-(3,4-dichlorophényl)- | 2-méthyl | 5-méthyl |
| 6-(4-fluorophényl)- | 2-méthyl | 4-méthyl |
| 6-(4-fluoro-3-méthylphényl)- | 2-méthyl | 4-chloro |
| 6-(4-fluorobenzyloxy)- | 2-chloro | 4-chloro |
| 6-(4-fluoro-3-méthylphényl)- | 2-chloro | 4-méthyl |

6. Le composé de la revendication 3 choisi parmi:

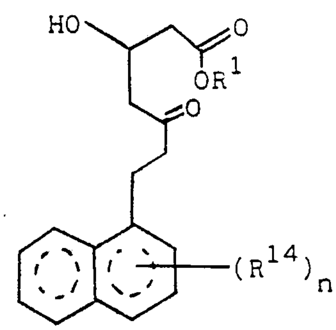

| n | $R^{14}$ | 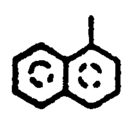 |
|---|---|---|
| 1 | 2-méthyl | naphtyle |
| 0 | - | naphtyle |
| 2 | 2,6-diméthyl | naphtyle |
| 1 | 2-méthyl | 5,6,7,8-tétrahydronaphtyle |

7. Une composition pharmaceutique antihypercholestérolémiante comprenant un support pharmaceutique et une quantité antihypercholestérolémiante efficace d'un composé comme revendiqué dans la revendication 1 ou 2.

8. La composition de la revendication 7, dans laquelle le composé antihypercholestérolémiant est comme revendiqué dans la revendication 3.

9. La composition de la revendication 8, dans laquelle le composé antihypercholestérolémiant est comme revendiqué dans les revendications 4, 5 ou 6.

10. Un procédé pour la préparation d'un composé répondant à la formule développée:

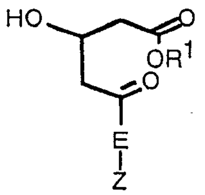

dans laquelle $R^1$, E et Z ont les significations de $R^1$, E et Z 1), 2) et 3) dans la revendication 1, qui comprend le traitement d'un composé répondant à la formule développée:

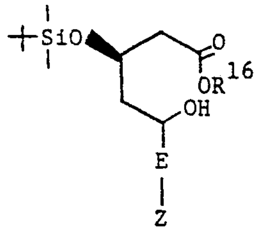

dans laquelle $R^{16}$ est un alkyle en $C_{1-4}$, avec un agent oxydant pour produire le composé de formule développée:

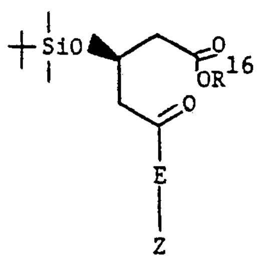

suivi d'une désilylation pour produire le composé de formule développée:

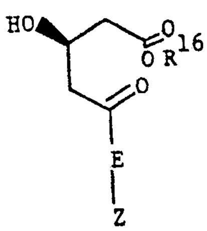

suivie d'un traitement avec un alcali pour former le produit dans lequel $R^{16}$ est un cation de métal alcalin, suivi d'une acidification pour produire le composé dans lequel $R^{16}$ est un ion hydrogène.

**Revendications pour l'Etat contractant: AT**

1. Un procédé pour la préparation d'un composé répondant à la formule développée:

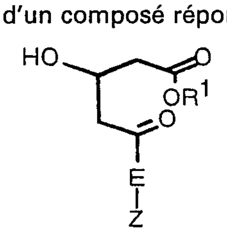

dans laquelle:

$R^1$ est

1) un hydrogène,

2) un alkyle en $C_{1-4}$,

3) un 2,3-dihydroxypropyle,

4) un cation de métal alcalin ou

5) un ammonium de formule $N^+R^3R^4R^5R^6$ dans laquelle $R^3$, $R^4$, $R^5$ et $R^6$ sont indépendamment un hydrogène ou un alkyle en $C_{1-4}$ ou deux de $R^3$, $R^4$, $R^5$ et $R^6$ sont réuinis pour former un hétérocycle à 5 ou 6 chaînons avec l'azote auquel ils sont fixés;

E est $-CH_2CH_2-$, $-CH=CH-$ ou $-(CH_2)_3-$; et

Z est

1)

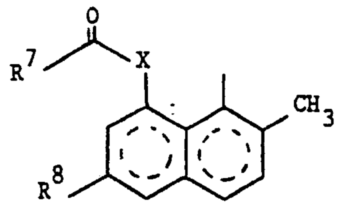

où les pointillés représentent tous les états d'oxydation possibles du système bicyclique;

X est —O— ou >$NR^9$ où $R^9$ est un hydrogène ou un alkyle en $C_{1-3}$;

$R^7$ est un alkyle en $C_{2-8}$; et

$R_8$ est un hydrogène ou $-CH_3$;

2)

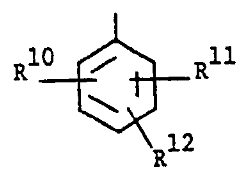

où $R^{10}$, $R^{11}$ et $R^{12}$ sont indépendamment

a) un hydrogène,

b) un halogène, tel que bromo, chloro ou fluoro,

c) un alkyle en $C_{1-4}$,

d) un halogénoalkyle en $C_{1-4}$,

e) un phényle soit non substitué soit substitué par un ou plusieurs de

i) alcoxy en $C_{1-4}$,

ii) alkyle en $C_{1-4}$,

iii) alcanoyloxy en $C_{2-8}$,

iv) halogénoalkyle en $C_{1-4}$ ou

v) halogéno,

f) $OR^{13}$ où $R^{13}$ est

i) un hydrogène,

ii) un alcanoyle en $C_{2-8}$,

iii) un benzoyle,

iv) un phényle,

v) un halogénophényle,

vi) un phényl-alkyle en $C_{1-3}$ soit non substitué soit substitué par un ou plusieurs halogènes, alcoxy en $C_{1-4}$, alkyles en $C_{1-4}$ ou halogénoalkyles en $C_{1-4}$,

vii) un alkyle en $C_{1-9}$,

viii) un cinnamyle,

ix) un halogénoalkyle en $C_{1-4}$,

x) un allyle,

xi) un cycloalkyl($C_{3-6}$)-alkyle en $C_{1-3}$,

xii) un adamantyl-alkyle en $C_{1-3}$,

3)

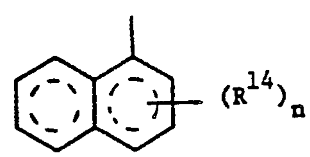

où n est 0—2 et $R^{14}$ est un halogéno ou alkyle en $C_{1-4}$, qui comprend le traitement d'un composé répondant à la formule développée:

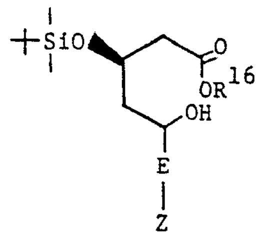

dans laquelle $R^{16}$ est un alkyle en $C_{1-4}$ avec un agent oxydant pour produire le composé de formule développée:

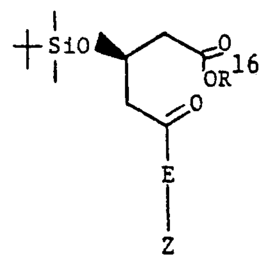

suivi d'une désilylation pour produire le composé de formule développée:

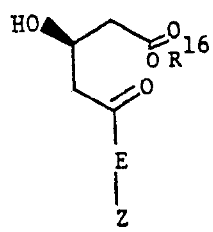

suivie d'un traitement avec un alcali pour former le produit dans lequel $R^{16}$ est un cation de métal alcalin, suivi d'une acidification pour produire le composé dans lequel $R^{16}$ est un ion hydrogène.

2. Un procédé pour la préparation d'un composé répondant à la formule développée:

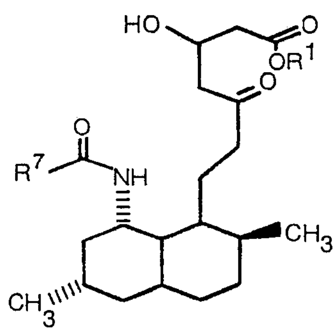

dans laquelle $R^1$ est un hydrogène, un cation de métal alcalin ou un cation ammonium et où $R^7$ est un 4-fluorobenzoyle, un 4-tert-butylbenzoyle ou un 4-fluorophénylacétyle, qui comprend le traitement d'un composé répondant à la formule développée:

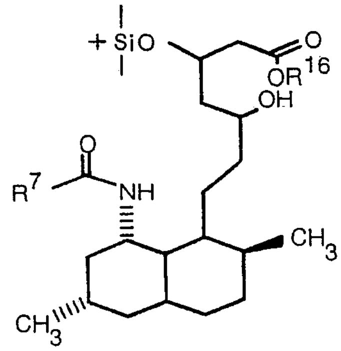

dans laquelle $R^{16}$ est un alkyle en $C_{1-4}$, avec un agent oxydant pour produire le composé de formule développée:

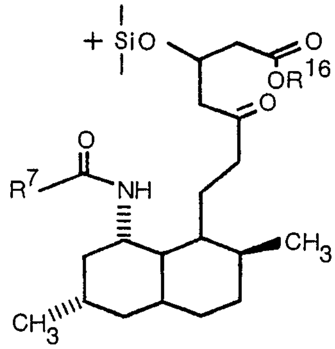

suivi d'une désilylation pour produire le composé de formule développée:

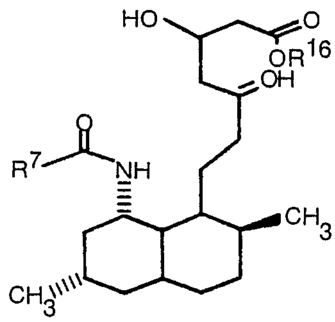

suivie d'un traitement avec un alcali pour produire le composé dans lequel $R^{16}$ est un cation de métal alcalin, suivi d'une acidification pour produire le composé dans lequel $R^{16}$ est un ion hydrogène.

3. Le procédé de la revendication 1 dans lequel:

$R^1$ est un hydrogène, un cation de métal alcalin ou un cation ammonium;

E est —CH=CH— ou —$CH_2CH_2$—; et

Z est

1)

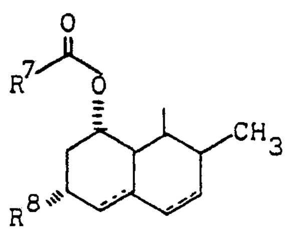

dans laquelle

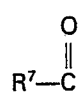

est un 2(S)-méthylbutyryle ou un 2-2-diméthylbutyryle;

2)

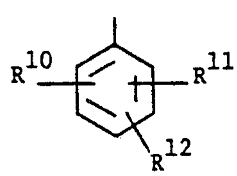

où $R^{10}$, $R^{11}$ et $R^{12}$ sont indépendamment:

a) un halogène,

b) un alkyle en $C_{1-4}$,

c) un halogénoalkyle en $C_{1-4}$,

d) un phényle ayant 1 à 3 substituants choisis parmi halogéno, alkyle en $C_{1-4}$ ou alcoxy en $C_{1-4}$,

e) $OR^{13}$, où $R^{13}$ est

i) un phényle,

ii) un halogénophényle, ou

iii) un phényl-alkyle en $C_{1-3}$ soit non substitué soit substitué par un ou plusieurs halogènes, alcoxy en $C_{1-4}$, alkyles en $C_{1-4}$ ou halogénoalkyles en $C_{1-4}$; ou

3) 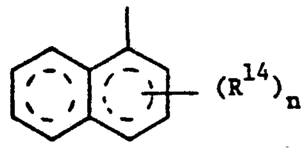

où n est 0, 1 ou 2 et $R^{14}$ est un méthyle, et le système cyclique est un naphtalène ou un 5,6,7,8-tétrahydronaphtalene.

4. Le procédé de la revendication 1 pour la préparation d'un composé choisi parmi:

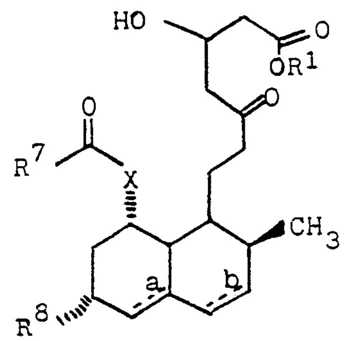

où $R^1$ est un hydrogène, un cation de métal alcalin ou un cation ammonium et $R^7CO$—, $R^8$, X, a et b ont les significations suivantes:

| $R^7\text{-}\overset{\text{O}}{\overset{\|}{\text{C}}}\text{-}$ | $R^8$ | X | a* | b |
|---|---|---|---|---|
| 2(S)-méthylbutyryle | $-CH_3$ | O | simple | double |
| 2(S)-méthylbutyryle | $-CH_3$ | O | simple | simple |
| 2(R)-méthylbutyryle | $-CH_3$ | O | double | double |
| 2,2-diméthylbutyryle | $-CH_3$ | O | double | double |
| 2,2-diméthylbutyryle | $-CH_3$ | O | simple | double |
| 2,2-diméthylbutyryle | $-CH_3$ | O | simple | simple |
| acétyle | $-CH_3$ | O | double | double |
| 2(S)-méthylbutyryle | H | O | double | double |
| 2(S)-méthylbutyryle | H | O | simple | simple |
| 2,2-diméthylbutyryle | H | O | double | double |
| 2,2-diméthylbutyryle | H | O | simple | simple |
| 2,2-diméthylbutyryle | $-CH_3$ | NH | simple | simple |
| 2-méthyl-2-éthylbutyryle | $-CH_3$ | NH | simple | simple |
| 2-méthylbutyryle | $-CH_3$ | NH | simple | simple |
| acétyle | $-CH_3$ | NH | double | double |
| acétyle | $-CH_3$ | $NCH_3$ | simple | simple |
| 2,2-diméthylbutyryle | $-CH_3$ | $NCH_3$ | simple | simple |
| 2,2-diméthylbutyryle | $-CH_3$ | NH | double | double |

* lorsque a = simple liaison, les cycles sont condensés en trans.

5. Le procédé selon la revendication 3 pour la préparation d'un composé choisi parmi

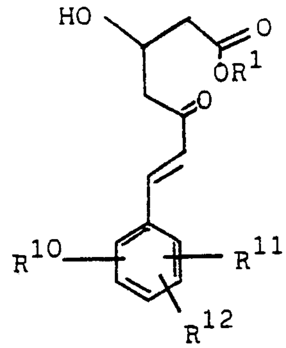

| $R^{10}$ | $R^{11}$ | $R^{12}$ |
|---|---|---|
| 6-(4-fluoro-3-méthylphényl)- | 2-méthyl | 4-méthyl |
| 6-(4-fluorophényl)- | 2-chloro | 4-chloro |
| 6-(4-chlorophényl)- | 2-chloro | 4-chloro |
| 6-(3,4-dichlorophényl)- | 2-chloro | 4-chloro |
| 6-(4-fluoro-3-méthylphényl)- | 2-chloro | 4-chloro |
| 6-(3,4-dichlorophényl)- | 2-méthyl | 4-méthyl |
| 6-(3,5-diméthylphényl)- | 2-chloro | 4-chloro |
| 6-(3,4-dichlorophényl)- | 2-méthyl | 5-méthyl |
| 6-(4-fluorophényl)- | 2-méthyl | 4-méthyl |
| 6-(4-fluoro-3-méthylphényl)- | 2-méthyl | 4-chloro |
| 6-(4-fluorobenzyloxy)- | 2-chloro | 4-chloro |
| 6-(4-fluoro-3-méthylphényl)- | 2-chloro | 4-méthyl |

6. Le procédé de la revendication 3 pour la préparation d'un composé choisi parmi:

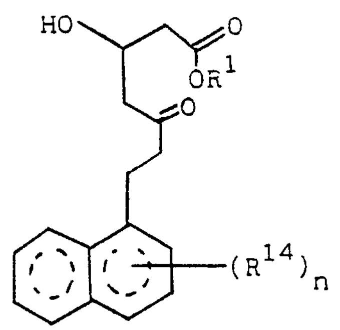

| n | $R^{14}$ | 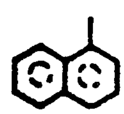 |
|---|---|---|
| 1 | 2-méthyl | naphtyle |
| 0 | - | naphtyle |
| 2 | 2,6-diméthyl | naphtyle |
| 1 | 2-méthyl | 5,6,7,8-tétrahydronaphtyle |

7. Un procédé pour la préparation d'un composé de formule:

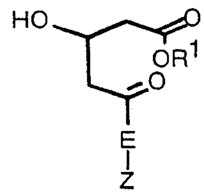

dans laquelle

$R^1$ est

1) un hydrogène,

2) un alkyle en $C_{1-4}$,

3) un 2,3-dihydroxypropyle,

4) un cation de métal alcalin ou

5) un ammonium de formule $N^+R^3R^4R^5R^6$ dans laquelle $R^3$, $R^4$, $R^5$ et $R^6$ sont indépendamment un hydrogène ou un alkyle en $C_{1-4}$ ou deux de $R^3$, $R^4$, $R^5$ et $R^6$ sont réunis pour former un hétérocycle à 5 ou 6 chaînons avec l'azote auquel ils sont fixés;

E est $-CH_2CH_2-$, ou $-(CH_2)_3-$; et

Z est

1) 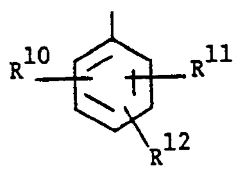

où $R^{10}$, $R^{11}$ et $R^{12}$ sont indépendamment

a) un hydrogène,

b) un halogène, tel que bromo, chloro ou fluoro,

c) un alkyle en $C_{1-4}$,

d) un halogénoalkyle en $C_{1-4}$,

e) un phényle soit non substitué soit substitué par un ou plusieurs de

i) alcoxy en $C_{1-4}$,

ii) alkyle en $C_{1-4}$,

iii) alcanoyloxy en $C_{2-8}$,

iv) halogénoalkyle en $C_{1-4}$ ou

v) halogéno,

f) $OR^{13}$ où $R^{13}$ est

i) un hydrogène,

ii) un alcanoyle en $C_{2-8}$,

iii) un benzoyle,

iv) un phényle,

v) un halogénophényle,

vi) un phényl-alkyle en $C_{1-3}$ soit non substitué soit substitué par un ou plusieurs halogènes, alcoxy en $C_{1-4}$, alkyles en $C_{1-4}$ ou halogénoalkyles en $C_{1-4}$,

vii) un alkyle en $C_{1-9}$,

viii) un cinnamyle,

ix) un halogénoalkyle en $C_{1-4}$,

x) un allyle,

xi) un cycloalkyl($C_{3-6}$)-alkyle en $C_{1-3}$ ou

xii) un adamantyl-alkyle en $C_{1-3}$,

2) 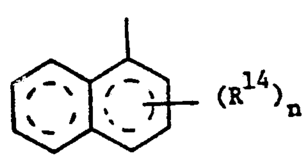

où n est 0—2 et $R^{14}$ est un halogéno ou un alkyle en $C_{1-4}$ qui comprend la réaction des composés:

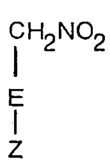 + 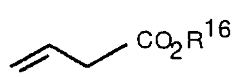

pour produire le composé de formule développée:

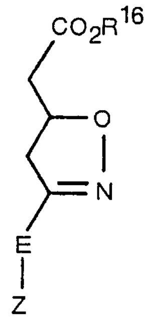

suivie d'une réduction catalytique pour produire le composé désiré dans lequel $R^1$ est $R^{16}$; suivie d'un traitement avec un alcali pour former le produit dans lequel $R^1$ est un cation de métal alcalin, suivi d'une acidification pour produire le composé dans lequel $R^1$ est un ion hydrogène.

8. Le procédé de la revendication 7, dans lequel:

$R^1$ est un hydrogène, un cation de métal alcalin ou un cation ammonium;

E est $-CH_2CH_2-$; et

Z est

1) 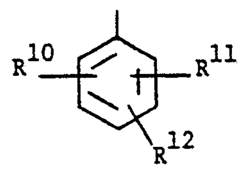

où $R^{10}$, $R^{11}$ et $R^{12}$ sont indépendamment:

a) un halogène,

b) un alkyle en $C_{1-4}$,

c) un halogénoalkyle en $C_{1-4}$,

d) un phényle avec 1 à 3 substituants choisis parmi halogéno, alkyle en $C_{1-4}$ ou alcoxy en $C_{1-4}$,

e) $OR^{13}$, où $R^{13}$ est

i) un phényle,

ii) un halogénophényle, ou

iii) un phényl-alkyle en $C_{1-3}$ soit non substitué soit substitué par un ou plusieurs halogènes, alcoxy en $C_{1-4}$, alkyles en $C_{1-4}$ ou halogénoalkyles en $C_{1-4}$; ou

2) 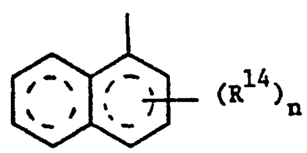

où n est 0, 1 ou 2 et $R^{14}$ est un méthyle et le système cyclique est un naphtalène ou un 5,6,7,8-tétrahydronaphtalène.

9. Le procédé de la revendication 8 pour la préparation d'un composé choisi parmi

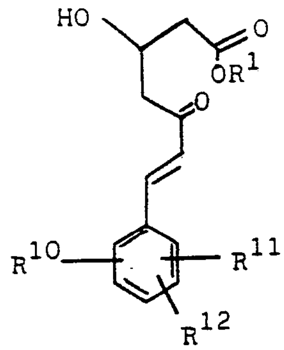

| $R^{10}$ | $R^{11}$ | $R^{12}$ |
|---|---|---|
| 6-(4-fluoro-3-méthylphényl)- | 2-méthyl | 4-méthyl |
| 6-(4-fluorophényl)- | 2-chloro | 4-chloro |
| 6-(4-chlorophényl)- | 2-chloro | 4-chloro |
| 6-(3,4-dichlorophényl)- | 2-chloro | 4-chloro |
| 6-(4-fluoro-3-méthylphényl)- | 2-chloro | 4-chloro |
| 6-(3,4-dichlorophényl)- | 2-méthyl | 4-méthyl |
| 6-(3,5-diméthylphényl)- | 2-chloro | 4-chloro |
| 6-(3,4-dichlorophényl)- | 2-méthyl | 5-méthyl |
| 6-(4-fluorophényl)- | 2-méthyl | 4-méthyl |
| 6-(4-fluoro-3-méthylphényl)- | 2-méthyl | 4-chloro |
| 6-(4-fluorobenzyloxy)- | 2-chloro | 4-chloro |
| 6-(4-fluoro-3-méthylphényl)- | 2-chloro | 4-méthyl |

10. Le procédé de la revendication 8 pour la préparation d'un composé choisi parmi:

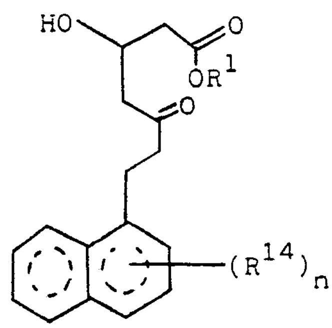

| n | $R^{14}$ | 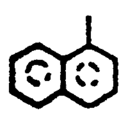 |
|---|---|---|
| 1 | 2-méthyl | naphtyle |
| 0 | - | naphtyle |
| 2 | 2,6-diméthyl | naphtyle |
| 1 | 2-méthyl | 5,6,7,8-tétrahydronaphtyle |

11. Un procédé pour la préparation d'un composé répondant à la formule développée:

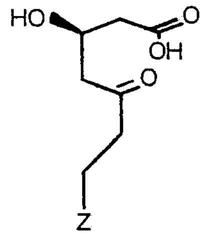

dans laquelle Z est comme défini dans la revendication 1, qui comprend le traitement d'un composé de formule développée:

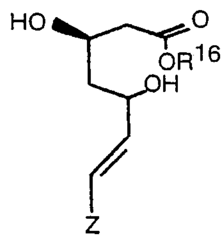

avec du dioxyde de manganèse activé pour produire le composé de formule développée:

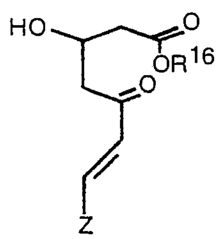

suivi d'un traitement avec l'hydrure de tri-n-butylétain et le tétrakis(triphénylphosphine)palladium(0).